# EUROPEAN PATENT APPLICATION

(11) **EP 3 689 373 A1**
(43) Date of publication of application: **05.08.2020**
(21) Application number: 19154466.7
(22) Date of filing: 30.01.2019
(51) Int. Cl.: A61K 39/102, C07K 14/285

(54) **INACTIVATED APXIA, APXIIA AND APXIIIA TOXINS**

(71) Applicant: IDT Biologika GmbH, 06861 Dessau-Rosslau (DE)
(72) Inventor: FLORIAN, Volker, 06862 Dessau-Rosslau (DE); PIDDE, Heiko, 39167 Niederndodeleben (DE); STENTZEL, Sebastian, 17491 Greifswald (DE)
(74) Representative: Isarpatent

(57) **Abstract**

Inactivated ApxIA, ApxIIA and ApxIIIA toxins, vaccines comprising said inactivated toxins and the use thereof for the immunization and protection of mammals are disclosed.

## Description

### FIELD OF THE INVENTION

The present invention relates to inactivated ApxIA, ApxIIA and ApxIIIA toxins, vaccines comprising said inactivated toxins and the use thereof for the immunization and protection of mammals.

### BACKGROUND OF THE INVENTION

*Actinobacillus pleuropneumoniae* (APP) is a Gram-negative bacterium and a member of the family Pasteurellaceae. APP is the etiological agent of porcine pleuropneumonia, a severe pulmonary disease of pigs that causes high economic losses in pig production worldwide. The disease is characterized by hemorrhagic, fibrinous and necrotic lung lesions. Pigs surviving the disease often become asymptomatic carriers of APP and are the main cause of bacterial dissemination.

To date, 18 serotypes have been identified on the basis of the antigenic properties of capsular polysaccharides (CPS) and as result of genotypic analysis. Main virulence factors of APP are exotoxins, CPS, lipopolysaccharides (LPS) and membrane proteins. The most important virulence factors are Apx-exotoxins which belong to the pore-forming repeats in toxin (RTX) family. The toxins are known to be highly immunogenic and are very important to obtain a protective immunity against APP related pleuropneumonia. At least four different Apx toxins are produced by APP, designated ApxI, ApxII, ApxIII and ApxIV. ApxI shows strong and Apxll shows low hemolytic activity. Both are cytotoxic and active against a broad range of cells of different host species. Apxlll is non hemolytic but strongly cytotoxic with porcine alveolar macrophages and neutrophils as major targets. ApxIV shows no cytotoxic and only week hemolytic activity. No serotype of APP produces all four Apx toxins. The majority of serotypes produce three Apx toxins. The pattern of Apx toxin production is associated with virulence, with serotypes 1, 5, 9 and 11 producing ApxI and Apxll being the most virulent.

At least four genes are responsible for production and secretion of active Apx toxins. Gene A encodes the structural toxin and gene C encodes an acyltransferase, mandatory for post translational activation of the toxin. Genes B and D encode two membrane proteins that are required for secretion of the mature toxin. The Apx genes are organized as operons. Operons of ApxI and ApxIII toxins consist of genes CABD whilst ApxII operon contains only the genes CA. Secretion of ApxIIA therefore depends on active genes B and D of the ApxI operon.

Presently, pleuropneumonia resulting from APP infection of pigs is usually treated with antibiotics. However, it has been found that APP often exhibits antibiotic resistance against at least one of the antibiotics commonly used to treat APP infection.

Vaccination against APP is a promising prophylactic strategy to prevent pleuropneumonia. Several vaccines have been commercialized. Commercially available vaccines are either chemically inactivated whole cell vaccines or subunit vaccines or a combination of both. The immunological reaction of animals vaccinated with whole cell vaccines is directed mainly against surface structures such as CPS and LPS. The absence of secreted proteins such as Apx toxins which are known to be highly immunogenic and essential for protection explains the limited protection observed with bacterins. Furthermore APP whole cell vaccines confer only homologous protection against the serotype used to prepare the vaccine.

The commercially available subunit vaccine (patent EP0453024 B1) contains chemically inactivated ApxA toxins as well as an outer membrane protein. The inactivation of ApxA toxins with denaturizing substances such as formaldehyde potentially leads to a decreased immunogenicity of the toxoids. The disadvantages of such a vaccine are the insufficient protection due to denaturizing toxins as well as concomitant serious side reactions probably due to remaining toxicity, due to incomplete inactivation of ApxA toxins. Subsequently, due to decreased immunogenicity after inactivation higher amounts of toxins need to be used that result in increased amounts of contaminating LPS in the vaccines. High LPS content can cause side effects as seen with commercial APP subunit vaccines.

It can be concluded, that current commercially available vaccines do not possess satisfactory safety and/or efficacy profiles against APP infection.

Furthermore there are some other experimental approaches for APP vaccines.

Patent application WO2004045639 (A1) discloses a live attenuated vaccine against porcine pleuropneumonia containing an APP strain which is modified in transmembrane domains of genes encoding the toxins ApxIA and ApxIIA. To test the degree of attenuation three months old pigs were inoculated with modified live APP strains. Seven days after the inoculation the animals were sacrificed and macroscopic lesions in the respiratory organs were recorded. All animals showed a modification of their behavior and lung lesions at necropsy. The efficacy of such a live vaccine was not examined.

Patent application EP0810283 (A2) and patent EP0861319 (B1) describe live attenuated APP strains having deletions in ApxA activator genes *apxC.* The modified APP strain does not produce the activator protein ApxC in a functional form and therefore the toxins ApxIA and ApxIIA were not activated by acylation. Mice were vaccinated with a ΔapxC strain and challenged with virulent APP field strains. Vaccinated mice were protected against homologous challenge and partially protected against heterologous challenge. One study in pigs was performed. 1 out of 6 vaccinated pigs had lung lesions after heterologous challenge and necropsy. These attenuated live vaccines seem to be efficacious but this vaccine bears a safety risk. The vaccine strains produce Apx toxins which are not activated by acylation, due to lacking apxC. However these toxins have the original amino acid sequence of toxic ApxA. Very likely heterologous acyltransferase can acylate the ApxA converting it into its active, toxic form. In most of pig farms worldwide there are asymptotic carriers of virulent APP strains as well as pigs infected with low virulent APP strains. If such pigs were vaccinated with a ΔapxC vaccine strain the non-activated ApxA toxins of the vaccine strain could be activated by functional ApxC proteins of field strains. Furthermore there is the possibility to complementation of the apxC deletion by uptake of functional apxC genes. There is the possibility that the attenuated strain revert to a pathogenic one resulting in disease of inoculated animals.

Until today no attenuated live vaccines are commercially available.

Accordingly, there is the need for improved vaccines against APP which are safe and able to induce cross protection against all relevant serotypes in pigs and/or young piglets.

It is therefore an object of the present invention to provide inactivated ApxIA, ApxIIA and ApxIIIA toxins and the use of these toxoids in safe and efficacious subunit and live vaccines against porcine pleuropneumonia.

### SUMMARY

The present invention discloses modifications of the acylation sides of the ApxIA, ApxIIA and ApxIIIA toxins to create inactive but fully immunogenic toxoid forms of these proteins. The replacement of both amino acids of the two acylation sides by another one prevents them from being acylated. Subsequently, these modified Apx toxins cannot initiate the binding to the target cell membrane and have no cytotoxic or haemolytic activity. The toxins do not need to be chemically inactivated resulting in highly immunogenic proteins. The required amount of these proteins for production of an efficacious subunit vaccine is relatively low. Therefore the content of contaminating LPS is also relatively low resulting in minor side effects such as fewer and vomiting and apathy. Due to better immunogenicity compared to chemically inactivated Apx subunits, subunit vaccines according to the present invention provide a better safety profile.

Furthermore, the invention can be used in live vaccines as the replacement of the two amino acids is based on genetic modification of the apxA genes. Thus, it can be transferred to APP strains that subsequently produce inactivated and highly immunogenic Apx proteins. These strains can be used as live vaccines which produce ApxA toxoids as well as a broad spectrum of other APP proteins. Those vaccines induce a complex immune response and cross protection against a broad range of APP serotypes. Due to genetic modification of the apxA genes the produced Apx toxoids cannot be activated by heterologous acyltransferases. There are some more advantages when using a live APP vaccine strain. The influence of maternal antibodies on the vaccine is expected to be lower when using live vaccines compared to subunit vaccines consisting of highly immunogenic antigens. Accordingly, with a high likelihood, the vaccine can be used in piglets before the age of 6 weeks and the existing immunological gap of available vaccines can be avoided. Additional gene knock outs can be used to achieve an even more attenuated vaccine strain and to use gene knock outs as marker for differentiating of infected from vaccinated animals (DIVA), e.g. by deletion of *apxIVA* gene. Hence, this invention provides a better safety and efficacy profile than all known vaccine approaches. As APP is strongly adapted to pigs, an attenuated APP vaccine strain could also be used as a vector for antigens of other pathogens that are relevant to pigs.

The invention is defined by the appended claims.

Disclosed are ApxIA, ApxIIA or ApxIIIA polypeptides of *Actinobacillus pleuropneumoniae* wherein
a. the wild type amino acid sequence of
   i. ApxIA as set forth SEQ ID NO:1 is modified in at least one amino acid selected from the group consisting of K560 and K686,
   ii. ApxIIA as set forth SEQ ID NO:2 is modified in at least one amino acid selected from the group consisting of K557 and N687, and
   iii. ApxIIIA as set forth SEQ ID NO: 3 is modified in at least one amino acid selected from the group consisting of K571 and K702,
   and the at least one amino acid is exchanged by an amino acid not susceptible to acylation; or
b. an analogue or a fragment of the modified amino acid sequence as defined under a. which is at least 90% homologous to the modified amino acid sequence as defined under a. and a fragment comprises at least 50% of the consecutive amino acids of the modified amino acid sequence as defined under a., and the analogue or fragment comprises the amino acid not susceptible to acylation as defined under a;
   or
c. the wild type amino acid sequence of
   i. ApxIA as set forth SEQ ID NO:1 containing deletions comprising at least one amino acid selected from the group consisting of K560 and K686,
   ii. ApxIIA as set forth SEQ ID NO:2 containing deletions comprising at least one amino acid selected from the group consisting of K557 and N687, and
   iii. ApxIIIA as set forth SEQ ID NO:3 containing deletions comprising at least one amino acid selected from the group consisting of K571 and K702,
   wherein the deletions do not delete more than 50% of the wild type amino acid sequence;
d. an analogue of the amino acid sequence comprising deletions as defined under c. which is at least 90% homologous to the amino acid sequence comprising deletions as defined under c.

### BRIEF DESCRIPTION OF THE FIGURES

Figure 1 illustrates the establishment of a cytotoxicity assay with BL-3 cells and ApxA toxins isolated from APP supernatants.
Figure 2 illustrates a cytotoxic assay with BL-3 cells and genetically modified ApxIA and wild type ApxIA toxin (A) and genetically modified ApxIIIA and wild type ApxIIIA toxin (B).
Figure 3 illustrates the establishment of a hemolytic assay with sheep blood and ApxA toxins isolated from APP supernatants.
Figure 4 illustrates a hemolytic assay with sheep blood and genetically modified ApxIA and wild type ApxIA toxin.

### DETAILED DESCRIPTION

The following headings merely serve to organize the disclosure, but should not be considered as separating one section of the disclosure from a further section of the disclosure. The features of each section can be combined with the features of any further section.

### GENERAL DEFINITIONS

The term "fragment or analogue" as used herein is defined as follows:
An "analogue" can be regarded as an amino acid sequence exhibiting a level of homology of at least 60%, 70%, 80%, 85%, 90%, 95% or 97% to the original amino acid sequence. Homology, as used herein, means identity. As such, the sequences might differ from each other based on conservative substitution, deletion or insertion.

The degree of identity can be determined with the protein blast program using the blastp algorithm with default parameters which are, for example, Expect threshold: 10, Word size: 3, Matrix: BLOMSUM62, Gap Costs: Existence: 11 Extension: 1 and Compositional adjustments: Conditional compositional score matrix adjustment (BLAST is a registered trademark of the National Library of Medicine). The program can be used to search a protein database using a protein query. Identity reports the exact matches between aligned query and database sequences.

Preferably, conservative amino acid "substitutions" are the result of replacing one amino acid with another amino acid having similar structural and/or chemical properties, i.e., conservative amino acid replacements. Amino acid substitutions may be made on the basis of similarity in polarity, charge, solubility, hydrophobicity, hydrophilicity, and/or the amphipathic nature of the residues involved. For example, nonpolar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid.

"Insertions" or "deletions" are typically in the range of about 1, 2 or 3 amino acids. The variation allowed may be experimentally determined by systematically introducing insertions or deletions of amino acids in a protein using recombinant DNA techniques and assaying the resulting recombinant variants for activity. This does not require more than routine experiments for a skilled person.

A "fragment" of a polypeptide comprises at least 50%, 60%, 70%, 80%, 90%, 95%, or 97% of the original polypeptide. These fragments may be used as the exclusive active ingredient in a vaccine according to the present invention.

An APP "toxin" is a polypeptide that consists of the amino acid sequence of ApxIA, ApxIIA, or ApIIIA (e.g. as set forth in SEQ ID NO: 1-3) and exhibits a cytotoxic and/or hemolytic activity. A "toxoid" in this disclosure is a polypeptide that is a modified form of the "toxin" wherein the modification is achieved by the replacement or deletion of an amino acid that is susceptible to acylation in vivo in APP and does not exhibit any cytotoxic or hemolytic activity.

The term "comprising" as used herein in the context of the vaccine composition means that further active or immunogenic components can be present. "Consisting of" means that no further components are present and "essentially consisting of" means that specific further components can be present, namely those not materially affecting the essential characteristics of the vaccine (i.e. inactive or not immunogenic ingredients).

The genus *Actinobacillus* comprises Gram-negative, nonspore-forming and predominantly encapsulated bacteria species that colonize the mucosal surface of the respiratory and urogenital tracts. Relevant veterinary species are for example *A. pleuropneumoniae, A. suis, A. equuli* and A. *lignieresii* which are the preferred Actinobacillus spp. of this disclosure . *Actinobacillus spp.* normally show strong host specificity. Preferred APP serotypes are serotypes 1, 5, 7, 8, 9 and 11.

### POLYPEPTIDES

The disclosure relates to ApxIA, ApxIIA or ApxIIIA polypeptides (hereafter exchangeably designated ApxA polypeptides for brevity) wherein the wildtype ApxIA, ApxIIA or ApxIIIA polypeptides have been modified by amino acid substitution or deletion at the position of the amino acids susceptible to acylation in the wild type polypeptide. The amino acid substitution introduces an amino acid that is not susceptible to acylation. It has been surprising found that ApxIA, ApxIIA or ApxIIIA polypeptides modified in the above described manner no longer exhibit any hemolytic and/or cytotoxic activity. Thus, they may exhibit less side effects when being used to vaccinated a mammal while conferring immunological protection against APP. Accordingly, they can be considered as inactivated toxins, i.e. toxoids. Therefore, the polypeptides of the disclosure can be used safely in vaccines for immunizing subjects, in particular, mammals, against APP infection.

### Polypeptides comprising substitutions of the position of the amino acids susceptible to acylation in the wild type polypeptide

In particular, the disclosure relates to an ApxIA, ApxIIA or ApxIIIA polypeptide of *Actinobacillus pleuropneumoniae* wherein
a. the wild type amino acid sequence of
   i. ApxIA as set forth SEQ ID NO: 1 is modified in at least one amino acid selected from the group consisting of K560 and K686,
   ii. ApxIIA as set forth SEQ ID NO: 2 is modified in at least one amino acid selected from the group consisting of K557 and N687, and
   iii. ApxIIIA as set forth SEQ ID NO: 3 is modified in at least one amino acid selected from the group consisting of K571 and K702,
   and the at least one amino acid is exchanged by an amino acid not susceptible to acylation.

The polypeptides can induce a humoral or /and cellular immunological response against one or more serotypes of APP in a mammal, in particular, a pig when being administered to said mammal. The polypeptides can induce a humoral or /and cellular immunological response against one strains of APP, for example, 1, 2, 3, 4, 5, 6, 7, 8, 9, or 10 strains of APP in a mammal, in particular, a pig when being administered to said mammal. Preferably, the polypeptides induce a sterile immunity against APP, APP strains, or APP serotypes.

Each of ApxIA, ApxIIA or ApxIIIA comprises two amino acids which are susceptible to acylation. The modifications can affect one or both said amino acids.

The disclosure also relates to an analogue or/and a fragment of the modified amino acid sequence as defined above which is at least 90% homologous to the modified amino acid sequence as defined above and a fragment comprises at least 50% of the consecutive amino acids of the modified amino acid sequence as defined above and the analogue or fragment comprises the amino acid not susceptible to acylation as defined above.

As defined above the analogue can differ from the wild type by deletions or conservative substitutions. However, the analogue always comprises those amino acids replacing the amino acids susceptible to acylation.

Since the modified polypeptide does not contain the amino acids susceptible to acylation the modified polypeptide cannot be acylated. Polypeptides modified in this way cannot be acylated by any endogenous or exogenous acyltransferase (e.g. an ApxC of *Actinobacillus pleuropneumoniae*). As experimentally shown below those modified polypeptides no longer exhibit substantial pathological effects and can thus be safely used in vaccine compositions. These modified polypeptides are safer to use in vaccine compositions than vaccines where the acyltransferase ApxC is deleted because they remain pathological even if a acyltransferase is provided exogenously, either by a naturally occurring strain of APP or any other source of an acyltransferase in vivo. The same considerations apply to the following polypeptides in which the one or two amino acids of APP which are susceptible to acylation are deleted.

### Polypeptides comprising deletions of the position of the amino acids susceptible to acylation

### in the wild type polypeptide

Instead of substituting the amino acids susceptible to acylation in the wild type polypeptide the amino acids can also be deleted to abolish the pathological effects of the wild type polypeptide.

Therefore, the disclosure also relates to an ApxIA, ApxIIA or ApxIIIA polypeptide of *Actinobacillus pleuropneumoniae* wherein the wild type amino acid sequence of
i. ApxIA as set forth SEQ ID NO:1 contains deletions comprising at least one amino acid selected from the group consisting of K560 and K686,
ii. ApxIIA as set forth SEQ ID NO:2 containing deletions comprising at least one amino acid selected from the group consisting of K557 and N687, and
iii. ApxIIIA as set forth SEQ ID NO:3 containing deletions comprising at least one amino acid selected from the group consisting of K571 and K702.

The polypeptides can induce a humoral or /and cellular immunological response against one or more serotypes of APP in a mammal, in particular, a pig when being administered to said mammal. The polypeptides can induce a humoral or /and cellular immunological response against one strains of APP, for example, 1, 2, 3, 4, 5, 6, 7, 8, 9, or 10 strains of APP in a mammal, in particular, a pig when being administered to said mammal. Preferably, the polypeptides induce a sterile immunity against APP, APP serotypes, APP strains, or APP serotypes,.

As stated above, each of ApxIA, ApxIIA or ApxIIIA comprises two amino acids which are susceptible to acylation. The deletion of said amino acids can comprise one or both of said amino acids.

Thus, the deletions can comprise point deletions where only either the one or the two amino acids susceptible to acylation in each wildtype sequence are deleted. It is also disclosed that the deletions also delete amino acids in an area adjacent to the one or two amino acids susceptible to acylation. Thus, the respective deletions may comprise a 2, 3, 4, 5, 6, 7, 8, 9, 10, 20, 30, 40, 60, 70, 80, 90, 100, 110, 120, 130, 140, 150, 200, 300, 350, 400 amino acids as long as the deletion comprises one of the two amino acids susceptible to acylation. The size of the deletion for each amino acid susceptible to acylation can be independent from each other of have the same size. Deletions which cover a consecutive stretch of amino acids between the two amino acids susceptible to acylation are also disclosed.

The deletions do not delete more than 50% of the wild type amino acid sequence.

The disclosure also relates to an analogue of the modified amino acid sequence as defined above which is at least 90% homologous to the modified amino acid sequence as defined above and the analogue does not comprise the amino acid not susceptible to acylation as defined above. Such an analogue can comprise conservative substitutions and deletions as defined in the section relating to the general definitions. Thus, the polypeptide may comprise further deletions outside the deleted region comprising the one or two amino acids susceptible to acylation, but these deletions will only delete 1, 2, 3 amino acids.

### AMINO ACIDS NOT BEING SUSCEPTIBLE TO ACYLATION

Amino acids susceptible to acylation are naturally occurring amino acids, e.g., lysine and/or asparagine. Amino acids not susceptible to acylation are known to the skilled person and can be used to substitute one or both of the amino acids susceptible to acylation. For example, the amino acid not susceptible to acylation can be independently selected from the group consisting of alanine, glycine, isoleucine, leucine, methionine, valine, serine, threonine, asparagine, glutamine, aspartic acid, histidine, aspartic acid, cysteine, proline, phenylalanine, tyrosine, tryptophan and glutamic acid. In particular, the amino acid not susceptible to acylation can be independently selected from the group consisting of alanine, glycine, serine, threonine, valine, isoleucine and leucine.

Moreover, the amino acid not susceptible to acylation can be independently selected from the group consisting of alanine, glycine, and serine. The most preferred amino acid amino acid not susceptible to acylation is alanine.

### APXIA, APXIIA OR APXIIIA POLYPEPTIDE WITH TWO MODIFIED AMINO ACIDS

Preferably, two amino acids susceptible to acylation are modified in each of ApxIA, ApxIIA or ApxIIIA.

Thus, the wild type sequence of ApxIA as set forth SEQ ID NO:1 is modified at K560 and K686. Thus, the wild type sequence of ApxIIA as set forth SEQ ID NO:2 is modified at K557 and N687. Thus, the wild type sequence of ApxIIIA as set forth SEQ ID NO: 3 is modified at K571 and K702.

Disclosed is also that said two amino acids susceptible to acylation in each of ApxIA, ApxIIA or ApxIIIA are modified to alanine.

Thus, the disclosed modified polypeptide can have the sequence set forth in SEQ ID NO: 4, 5, or 6 or be an analog or fragment thereof as defined above.

### NUCLEIC ACIDS

Nucleic Acids comprising a nucleic acid sequence capable of coding for the above described polypeptides are also disclosed. The disclosed nucleic acid can be cDNA, DNA, RNA, cRNA or PNA (peptide nucleic acid). The term "nucleic acid sequence" refers to a heteropolymer of nucleotides of the sequence of these nucleotides. The nucleic acid can comprise a nucleic acid as set forth in SEQ ID NO: 10, 11, or 12.

The nucleic acid can be comprised in a vector suitable for cloning or expressing the nucleic acids of the disclosure. Exemplary vectors are pEX-A258 (SEQ ID NO: 19), pQE-80L (SEQ ID NO: 20), or pQE-60 (SEQ ID NO: 21). The nucleic acids or vectors may comprise additional regulatory non coding elements like inducible or non-inducible promoters, operators (e.g. lac-operator) or nucleic acids coding for other AP proteins. Further it is preferred that a vaccine composition, according to the present invention, is further characterized by the polynucleotide integrated into a vector, wherein the polynucleotide is operably linked to an expression control region of the vector. Thus, expression vector are also disclosed, wherein the expression vector preferably comprises one or more regulatory sequences in addition to the nucleic acid sequences coding for modified forms of ApxA. The term "expression vector" generally refers to a plasmid or phage or virus or vector, for expressing a polypeptide from a DNA (RNA) sequence. An expression vector can comprise a transcriptional unit comprising an assembly of (1) a genetic element or elements having a regulatory role in gene expression, for example, promoters or enhancers, (2) a structural or coding sequence which is transcribed into mRNA and translated into protein, and (3) appropriate transcription initiation and termination sequences. Structural units intended for use in yeast or eukaryotic expression systems preferably include a leader sequence enabling extracellular secretion of translated protein by a host cell. Alternatively, where recombinant protein is expressed without a leader or transport sequence, it may include an N-terminal methionine residue. This residue may or may not be subsequently cleaved from the expressed recombinant protein to provide a final product.

### MICROORGANISMS

Also disclosed are microorganisms comprising the disclosed nucleic acids or/and vectors described above. The microorganism may be an *Escherichia coli* (*E. coli*), an *E. coli* strain, e.g. *E. coli* Top10F' strain or *Actinobacillus pleuropneumoniae,* an *Actinobacillus pleuropneumoniae* strain, e.g. ST2 (e.g. APP23), ST2 (e.g. 07/07), ST5 (e.g. DZY47), ST7 (e.g. DZY33), or ST8 (e.g. DZY49). The microorganisms also comprise nucleic acids or/and vectors coding for other genes.

### VACCINE COMPOSITION

Disclosed is also a vaccine composition comprising at least one modified ApxIA, ApxIIAor ApxIIIA polypeptide as defined above, the nucleic acid molecule as defined above or the vector as defined above, or a microorganism as defined above. The vaccine composition may comprise at least a pharmaceutical carrier, a diluent, or/and an adjuvant.

The preparation of the vaccine composition according to the invention is known in the art, and is described in handbooks known to the person skilled in the art. For the production of the vaccine composition according to the present invention pharmaceutically acceptable carriers, diluents or adjuvants which comprise but are not limited to the following: mineral salt adjuvants (e.g., alum-, calcium-, iron-, zirconium-based), tensoactive adjuvants (e.g., Quil A, QS-21, other saponins), bacteria-derived adjuvants (e.g., N-acetyl muramyl-L-alanyl-D-isoglutamine (MDP), lipopolysaccharides (LPS), monophosphoryl lipid A, trehalose dimycolate (TDM), DNA, CpGs, bacterial toxins), adjuvant emulsions (e.g., FIA, Montanide, Adjuvant 65, Lipovant), liposome adjuvants, polymeric adjuvants and carriers, cytokines (e.g., Granulocyte-macrophage colony stimulating factor), carbohydrate adjuvants, living antigen delivery systems (e.g., bacteria especially modified APP). Furthermore carriers can also comprise dry formulations such as coated patches made from titan or polymer. Techniques for formulation and administration of the vaccines of the present application may also be found in "Remington, The Science and Practice of Pharmacy", 22nd edition.

The vaccine composition as a unit composition may comprise 0.01 -2.0 mg of protein, 0.01 -2.0 mg of nucleic acid, or 0.5 -200 mg (or 1x10⁴ CFU-1x10¹⁰CFU, colony forming units) of microorganism. The required active amount of the protein, nucleic acid or microorganisms can be determined by routine testing methods by the skilled person, e.g. in pigs or piglets.

The vaccine composition comprising the at least one ApxIA, ApxIIA or ApxIIIA polypeptide as described above may be a subunit vaccine. The subunit vaccine may comprise further APP polypeptides.

It also contemplated that the vaccine substantially only contains a nucleic acid or vector, for example, is a DNA vaccine. DNA vaccines are third generation vaccines. Nucleic acid or DNA vaccines contain DNA/nucleic acids that codes for specific proteins (ApxAs) from APP. The DNA/nucleic acid is injected into the body of the mammal and taken up by cells, whose normal metabolic processes synthesize proteins based on the genetic code in the nucleic acid that they have taken up. Because these proteins contain regions of amino acid sequences that are characteristic of APP, they are recognized as foreign and when they are processed by the host cells and displayed on their surface, the immune system is alerted, which then triggers immune responses.

Alternatively, the nucleic acid may be encapsulated in protein to facilitate cell entry. If this capsid protein is included in the nucleic acid, the resulting vaccine can combine the potency of a live vaccine without reversion risks.

The microorganism as described above in the vaccine composition may be an *Actinobacillus species, for example, Actinobacillus pleuropneumoniae, Actinobacillus suis,* an *Actinobacillus species* strain, for example, a strain of *Actinobacillus pleuropneumoniae, Actinobacillus suis, or a particular serotype of a strain of an Actinobacillus species.* The vaccine composition may comprise at least 1, for example, 1, 2, 3, 4, 5, 6, or 7 *Actinobacillus species* strains or serotypes of an *Actinobacillus species strain.* Each of the *Actinobacillus species, strains, or serotypes* may code for at least one polypeptide as defined above.

The microorganism as described above in the vaccine composition may not comprise APP nucleic acids coding for APP proteins that are different from the disclosed modified ApxIA, ApxIIA or ApxIIIA polypeptides. For example, the microorganism may comprise deletions of the the apxIV gene or/and the sxy gene.

In addition, at least one of the *Actinobacillus pleuropneumoniae* in the vaccine composition may comprise one or at least two of the following modifications (e.g. single or multiple deletions): ΔtbpA, ΔtonB2, ΔsodC, ΔdsbA, Δfur, ΔmlcA, ΔmglA, ΔexbB, ΔureC, double mutant ΔexbB ΔureC, ΔfhuA, ΔhlyX, double mutant ΔapxIC and ΔapxIIC, triple mutant ΔapxIC ΔapxIIC Δorf1, hexa mutant ΔapxIIA ΔureC ΔdmsA ΔhybB ΔaspA Δfur, double mutant ΔapIIIB ΔapxIIID, double mutant ΔclpP and ΔapxIIC, ΔznuA, ΔapfA, double mutant ΔapxIIA and ΔureC, penta mutant ΔapxIC ΔapxIIC Δorf1 ΔcpxAR ΔarcA, double mutant ΔapxIC ΔompP2, double mutant ΔapxIIC ΔapxIVA, inactivated apxIIC,, inactivated apxIC, Δlip40, ΔcpxA/cpxR, ΔpotD2, ΔtoIC2, ΔsapA, or ΔPdxS/PdxT.

The above modifications are disclosed in:
Baltes, Nina; Hennig-Pauka, Isabel; Gerlach, Gerald-F (2002): Both transferrin binding proteins are virulence factors in Actinobacillus pleuropneumoniae serotype 7 infection. In FEMS microbiology letters 209 (2), pp. 283-287,
Sheehan, B. J.; Bosse, J. T.; Beddek, A. J.; Rycroft, A. N.; Kroll, J. S.; Langford, P. R. (2003):
   Identification of Actinobacillus pleuropneumoniae Genes Important for Survival during Infection in Its Natural Host. In Infection and immunity 71 (7), pp. 3960-3970. DOI: 10.1128/IAI.71.7.3960-3970.2003,
Baltes, N.; Tonpitak, W.; Gerlach, G. F.; Hennig-Pauka, I.; Hoffmann-Moujahid, A.; Ganter, M.; Rothkötter, H. J. (2001): Actinobacillus pleuropneumoniae iron transport and urease activity. Effects on bacterial virulence and host immune response. In Infection and immunity 69 (1), pp. 472-478. DOI: 10.1128/IAI.69.1.472-478.2001,
Jacques, Mario (2004): Surface polysaccharides and iron-uptake systems of Actinobacillus pleuropneumoniae. In Canadian journal of veterinary research = Revue canadienne de recherche veterinaire 68 (2), pp. 81-85,
Baltes, Nina; N'diaye, Mohamed; Jacobsen, IIse D.; Maas, Alexander; Buettner, Falk F. R.; Gerlach, Gerald-F (2005): Deletion of the anaerobic regulator HlyX causes reduced colonization and persistence of Actinobacillus pleuropneumoniae in the porcine respiratory tract. In Infection and immunity 73 (8), pp. 4614-4619. DOI: 10.1128/IAI.73.8.4614-4619.2005,
Lin, Liwen; Bei, Weicheng; Sha, Yonggang; Liu, Jinlin; Guo, Yi; Liu, Weihong et al. (2007): Construction and immunogencity of a DeltaapxIC/DeltaapxIIC double mutant of Actinobacillus pleuropneumoniae serovar 1. In FEMS microbiology letters 274 (1), pp. 55-62. DOI: 10.1111/j.1574-6968.2007.00813.x,
Yuan, Fangyan; Liu, Jinlin; Guo, Yi; Tan, Chen; Fu, Shulin; Zhao, Jin et al. (2011): Influences of ORF1 on the virulence and immunogenicity of Actinobacillus pleuropneumoniae. In Current microbiology 63 (6), pp. 574-580. DOI: 10.1007/s00284-011-0016-0,
Maas, Alexander; Jacobsen, IIse D.; Meens, Jochen; Gerlach, Gerald-F (2006): Use of an Actinobacillus pleuropneumoniae multiple mutant as a vaccine that allows differentiation of vaccinated and infected animals. In Infection and immunity 74 (7), pp. 4124-4132. DOI: 10.1128/IAI.00133-06,
Park, Changbo; Ha, Yooncheol; Kim, Soohee; Chae, Chanhee; Ryu, Doug-Young (2009): Construction and characterization of an Actinobacillus pleuropneumoniae serotype 2 mutant lacking the Apx toxin secretion protein genes apxIIIB and apxIIID. In The Journal of veterinary medical science 71 (10), pp. 1317-1323,
Xie, Fang; Li, Gang; Zhou, Long; Zhang, Yanhe; Cui, Ning; Liu, Siguo; Wang, Chunlai (2017): Attenuated Actinobacillus pleuropneumoniae double-deletion mutant S-8ΔclpP/apxIIC confers protection against homologous or heterologous strain challenge. In BMC veterinary research 13 (1), p. 14. DOI: 10.1186/s12917-016-0928-9,
Yuan, Fangyan; Liao, Yonghong; You, Wujin; Liu, Zewen; Tan, Yongqiang; Zheng, Chengkun et al. (2014): Deletion of the znuA virulence factor attenuates Actinobacillus pleuropneumoniae and confers protection against homologous or heterologous strain challenge. In Veterinary microbiology 174 (3-4), pp. 531-539. DOI: 10.1016/j.vetmic.2014.10.016,
Zhou, Yang; Li, Lu; Chen, Zhaohui; Yuan, Hong; Chen, Huanchun; Zhou, Rui (2013): Adhesion protein ApfA of Actinobacillus pleuropneumoniae is required for pathogenesis and is a potential target for vaccine development. In Clinical and vaccine immunology: CVI 20 (2), pp. 287-294. DOI: 10.1128/CVI.00616-12,
Tonpitak, Walaiporn; Baltes, Nina; Hennig-Pauka, Isabel; Gerlach, Gerald F. (2002): Construction of an Actinobacillus pleuropneumoniae serotype 2 prototype live negative-marker vaccine. In Infection and immunity 70 (12), pp. 7120-7125,
Yuan, Fangyan; Liu, Jinlin; You, Wujin; Bei, Weicheng; Wang, Chunlai; Zhao, Jin et al. (2018): Generation, safety and immunogenicity of an Actinobacillus pleuropneumoniae quintuple deletion mutant SLW07 (ΔapxICΔapxIICΔorf1ΔcpxARΔarcA). In Vaccine 36 (14), pp. 1830-1836. DOI: 10.1016/j.vaccine.2018.02.083,
Liu, Qiong; Gong, Yuheng; Cao, Yuqin; Wen, Xintian; Huang, Xiaobo; Yan, Qigui et al. (2013): Construction and immunogenicity of a ΔapxIC/ompP2 mutant of Actinobacillus pleuropneumoniae and Haemophilus parasuis. In The Onderstepoort journal of veterinary research 80 (1), p. 519. DOI: 10.4102/ojvr.v80i1.519,
Liu, Jinlin; Chen, Xia; Lin, Liwen; Tan, Chen; Chen, Yan; Guo, Yi et al. (2007): Potential use an Actinobacillus pleuropneumoniae double mutant strain DeltaapxIICDeltaapxIVA as live vaccine that allows serological differentiation between vaccinated and infected animals. In Vaccine 25 (44), pp. 7696-7705. DOI: 10.1016/j.vaccine.2007.07.053,
Bei, Weicheng; He, Qigai; Yan, Lin; Fang, Liurong; Tan, Yadi; Xiao, Shaobo et al. (2005): Construction and characterization of a live, attenuated apxIICA inactivation mutant of Actinobacillus pleuropneumoniae lacking a drug resistance marker. In FEMS microbiology letters 243 (1), pp. 21-27. DOI: 10.1016/j.femsle.2004.11.033,
Xu, Fu-Zhou; Shi, Ai-Hua; Chen, Xiao-Ling; Yang, Bing; Wang, Jin-Luo (2007): Construction and immunogenicity of an attenuated mutant of Actinobacillus pleuropneumoniae by insertional inactivation of apxIC. In Wei sheng wu xue bao = Acta microbiologica Sinica 47 (5), pp. 923-927, Prideaux, C. T.; Lenghaus, C.; Krywult, J.; Hodgson, A. L. (1999): Vaccination and protection of pigs against pleuropneumonia with a vaccine strain of Actinobacillus pleuropneumoniae produced by site-specific mutagenesis of the Apxll operon. In Infection and immunity 67 (4), pp. 1962-1966,
Liu, 2018 Outer Membrane Lipoprotein Lip40 Modulates Adherence, Colonization, and Virulence of Actinobacillus pleuropneumoniae. Front Microbiol. 2018 Jul 3;9:1472. doi: 10.3389/fmicb.2018.01472,
Li, 2018 The CpxA/CpxR Two-Component System Affects Biofilm Formation and Virulence in Actinobacillus pleuropneumoniae. Front Cell Infect Microbiol. 2018 Mar 20;8:72. doi: 10.3389/fcimb.2018.00072,
Zhu 2017 Polyamine-binding protein PotD2 is required for stress tolerance and virulence in Actinobacillus pleuropneumoniae. Antonie Van Leeuwenhoek. 2017 Dec;110(12):1647-1657. doi: 10.1007/S10482-017-0914-7,
Li 2017 TolC2 is required for the resistance, colonization and virulence of Actinobacillus pleuropneumoniae. J Med Microbiol. 2017 Jul 31. doi: 10.1099/jmm.0.000544,
Xie 2017 The SapA Protein Is Involved in Resistance to Antimicrobial Peptide PR-39 and Virulence of Actinobacillus pleuropneumoniae. Front Microbiol. 2017 May 10;8:811. doi: 10.3389/fmicb.2017.00811, and
Xie 2017 Pyridoxal phosphate synthases PdxS/PdxT are required for Actinobacillus pleuropneumoniae viability, stress tolerance and virulence. PLoS One. 2017 Apr 27;12(4):e0176374. doi: 10.1371/journal.pone.0176374.
It is expected that the combination of the modified polypeptides as disclosed herein with the additional known modifications listed above will result in a synergistic attenuation of *Actinobacillus pleuropneumoniae.*

### MEDICAL USE OR METHOD

The disclosed vaccine composition can be used in the prophylactic, metaphylactic or therapeutic treatment of a pneumonia, a pleurisy or a pleuropneumonia, in particular, of a pneumonia, a pleurisy or a pleuropneumonia caused by *Actinobacillus pleuropneumoniae* in a subject. The subject to be treated can be a mammal, in particular, a pig. The vaccine composition can be administered intramuscularly, intradermally, intravenously, subcutaneously, or by mucosal application (e.g. intranasally).

The vaccine composition can be administered via at least one, for example, one or two administrations using a unit composition as described above. In particular, the composition can be administered for the first time on the day of the birth, within 3 days, 1 week, 2 weeks, 4 weeks, 6 weeks, 8 weeks, 10 weeks or 12 weeks of the birth of the subject. Accordingly, the vaccine can be advantageously administered at an early point in time of the mammal. However, it is also disclosed that the vaccine can be administered at any time point of the life of the mammal.

The vaccine composition can be administered for a second time, wherein the time period between two administrations can be between 1 and 4 weeks, 1 and 3 weeks, or 1 and 2 weeks. Preferably, a vaccine composition comprising a microorganism is to be administered only once.

### EXAMPLES

The following examples provide experimental data explain the present invention and shall only be understood as illustrating the invention. In particular, it contemplated that each specific feature disclosed in the examples can be combined with the features of the anteceding part of the description.

### EXAMPLE 1: CLONING OF WILD TYPE AND MODIFIED APXIA, APXIIA, OR APXIIIA IN PEX-A258

Protein sequences of the wild types of ApxIA-ApxIIIA were obtained from the data base uniprot, i.e. ApxIA APP ST5b (uniprot: A3N292), ApxIIA APP ST3 (uniprot BOBPPO, with modification S148G), and ApxIIIA ST8 (uniprot: P55131).

In addition, protein sequences were provided comprising the following modifications:

**Table 1**

| | Acylation site 1 | Acylation site 2 |
|---|---|---|
| ApxIA (110 kDa) | K560A | K686A |
| ApxIIA (102 kDa) | K557A | N687A |
| ApxIIIA (112 kDa) | K571A | K702A |

The nucleic acids were derived from the protein sequences wherein the nucleic acid sequences were codon optimized and synthetically manufactured (Eurofins Genomics) without the start codon. Each nucleic acid sequence was provided in plasmid pEX-A258 (SEQ ID NO: 19) flanked by restriction sites BamHI at the 5' end and Sacl at the 3' end.

### EXAMPLE 2: CLONING AND EXPRESSION OF WILD TYPE AND MODIFIED APXIA-APXIIIA IN PQE-80L (SEQ ID NO: 20)

1 µg of pEX-A258 comprising the respective ApxIA-ApxIIIA was restricted with BamHI FD (ThermoFisher) and Sacl FD (ThermoFisher) according to the instructions of the manufacturer and resolved over a 1% agarose gel. Nucleic acid fragments of the appropriate size were extracted from the agarose gel (QUIAEX-II). pQE-80L was restricted with BamHI FD (ThermoFisher) and Sacl FD (ThermoFisher) according to the instructions of the manufacturer. Ligation of pQE-80L and the respective nucleic acid fragment in a ratio of 1:5 was performed over night at room temperature using T4-DNA ligase (ThermoFisher). The ligated DNA was electroporated into *E. coli* Topf10F' and the successful cloning of wildtype and modified forms of ApxIA-ApxIIIA was confirmed by restriction analysis and sequencing. pQE-80L comprises a ampicillin resistance.

### EXAMPLE 3: CLONING OF APXIC, APXIIC, AND APXIIIC GENES IN PQE-60

*apxIC-IIIC* genes were amplified from APP-DNA via polymerase chain reaction (PCR) using the primers indicated in table 2 and subsequently purified.

The PCR fragments comprising the respective *apxIC-IIIC* nucleic acids and pQE-60 were restricted with the restriction enzymes indicated in table 2 below according to the instructions of the respective manufacturer and resolved over a 1% agarose gel.

**Table 2**

| primer oder plasmid | Sequence | Restriction enzyme |
|---|---|---|
| apxIc_for_pqe60 | 5' GCA**TCATGA**GTAAAAAAATTAATGGATTTGAGG (SEQ ID NO: 23) | PagI |
| apxIc_rev_pqe60 | 5' GGT**AGATCT**TTAGCTATTTACTAATGAAAATTTAA (SEQ ID NO: 24) | BglII |
| apxIIc_for_pqe60 | 5' GCA**ACATGT**TAAAAAATGATTTTAACGTATTGG (SEQ ID NO: 25) | PciI |
| apxIIc_rev_pqe60 | 5' GGT**AGATCT**TTATTGTAGAGCTGTTATTAACT (SEQ ID NO: 26) | BglII |
| apxIIIc_for_pqe60 | 5' GCA**TCATGA**GTTATAAAAATGTTAAAAATTTAACA (SEQ ID NO: 27) | PagI |
| apxIIIc_rev_pqe60 | 5' GGT**AGATCT**TTATCCTCTTACAATTTTTTGTG (SEQ ID NO: 28) | BglII |
| pQE-60 | (SEQ ID NO: 21) | NcoI and BglII |

Nucleic acid fragments of the appropriate size were extracted from the agarose gel (QUIAEX-II). Ligation of pQE-60 and the respective nucleic acid fragment in a ratio of 1:5 was performed over night at room temperature using T4-DNA ligase (ThermoFisher).

The ligated DNA was electroporated into *E. coli* Topf10F' and the successful cloning was confirmed by restriction analysis and sequencing.

### EXAMPLE 4: CLONING AND EXPRESSION OF APXIC, APXIIC, AND APXIIIC GENES IN PACYC184

The respective *apxIC, apxIIC, and apxIIIC* genes were amplified from the respective pQE-60 vectors of example 2 using the primers indicated in table 3 below.

**Table 3**

| primer oder plasmid | Sequence | Restriction enzyme |
|---|---|---|
| apxIc_for_pacyc184 | 5' GCA**TCTAGA**AAATCATAAAAAATTTATTTGCTTT (SEQ ID NO: 29) | XbaI |
| apxIc_rev_pacyc184 | 5' AGC**TCGCGA**TTAGCTATTTACTAATGAAAATTTA (SEQ ID NO: 30) | NruI |
| apxIIc_for_pacyc184 | 5' GCA**ATCGAT**AAATCATAAAAAATTTGCTTT (SEQ ID NO: 31) | ClaI/BscI |
| apxIIc_rev_pacyc184 | 5' AGC**TCGCGA**TfATTGTAGAGCTGTfATfAACT (SEQ ID NO: 32) | NruI |
| apxIIIc_for_pacyc184 | 5' GCA**TCTAGA**AAATCATAAAAAATTTATTTGCTTT (SEQ ID NO: 33) | XbaI |
| apxIIIc_rev_pacyc184 | 5' AGC**TCGCGA**TTATCCTCTTACAATTTTTTTGT (SEQ ID NO: 34) | NruI |
| pACYC184 | (SEQ ID NO: 22) | XbaI and Nrul (*apxIC* and *apxIIIC*); |
| | | ClaI/BscI and NruI (*apxIIC*) |

In this way the promoter/lac-operator and the ribosomal binding site were also contained in the amplicons (SEQ ID NO: 16, 17, and 18).

The respective PCR products were restricted using the restriction enzymes indicated in table 3 and cloned into pACYC184 via restriction sites Xbal and Nrul (*apxIC* and *apxIIIC*) or Clal / Bscl and Nrul (*apxIIC*) using the same method and conditions as indicated above. pACYC184 comprises a chloramphenicol resistance.

The ligated DNA was electroporated into *E. coli* Topf10F' and the successful cloning was confirmed by restriction analysis and sequencing.

### EXAMPLE 5: CO-TRANSFORMATION OF PACYC184 COMPRISING APXIC, APXIIC, OR APXIIIC GENES AND PQE-80L COMPRISING APXIA, APXIIA, OR APXIIIA

ApxC is required for the activation of ApxA toxins. Accordingly, pACYC184 comprising the respective *apxIC, apxIIC,* or *apxIIIC* genes obtained in example 4 were cotransformed with a pQE-80L comprising the respective *apxIA, apxIIA,* or *apxIIIA* gene into *E. coli* Topf10F' and successful co-transformation was selected on LB agar plates comprising 50 µg/ml ampicillin and 15 µg/ml chloramphenicol. Successful transformation was confirmed by restriction analysis.

### EXAMPLE 6: EXPRESSION AND ISOLATION OF APXA, IN PARTICULAR, APXIA, TOXINS AND TOXOIDS

An *E. coli* colony as obtained in Example 5 and comprising pACYC184 and pQE-80L coding for the respective ApxA and ApxC genes was used to inoculate 40 ml LB-medium (100 µg/ml ampicillin and 30 µg/ml chloramphenicol). The culture was incubated under shaking at 30°C over night. On the next day the overnight culture was inoculated in 400 ml LB medium (100 µg/ml ampicillin and 30 µg/ml chloramphenicol) such that at optical density of 0.1 (optical density at 600 nm = OD600) was achieved. The obtained culture was incubated under shaking until an OD600 of 0.5 was obtained. To induce expression of recombinant protein 1 mM IPTG (Isopropyl β-D-1-thiogalactopyranoside) was added. The culture was incubated under shaking at 30°C for an additional 4 hours. Subsequently, the culture was centrifuged at 7000 g for 10 minutes. After removal of the supernatant the bacteria pellet was resuspended in LEW buffer (50 mM NaH₂PO₄, 300 mM NaCl in Aqua dest.; pH 8.0) with 5 ml LEW buffer per 1 g bacteria pellet and stored at -20°C.

1 ml 10x Bug Buster (Novagen), 5 µl Benzoase (25 U/µl, Novagen) and 10 mg lysozyme were added to 10 ml of resuspended bacteria pellet. The obtained suspension was rotated slowly at room temperature for 20 minutes. After centrifugation at 4°C and 15000 g for 60 minutes the pellet was washed in LEW buffer (10 ml LEW buffer per 1 mg of pellet) and centrifuged (4 °C, 15000 g, 60 min). The washed pellet was resuspended in 2 ml denaturing solubilization buffer (8 M urea in LEW buffer; pH=8.0) per 1 g of pellet and subsequently 1 ml Bug Buster (Novagen) per 10 ml of resuspended pellet. The suspension was incubated at 4°C for 1 hour and subsequently centrifuged (4 °C, 18000 g, 15 min). The protein containing supernatant was utilized in the subsequent steps for column protein purification.

According to the instructions of the manufacturer the columns (Protino Ni-IDA 2000 columns, Machery-Nagel) were equilibrated with 4 ml of equilibration buffer (8 M urea in LEW buffer; pH=8.0). Subsequently, the column was loaded with half of the protein containing supernatant and washed twice with 4 ml of equilibration buffer (total 8 ml buffer). Proteins were eluted twice with 3 ml elution buffer (equilibration buffer with 250 mM imidazol, total 6 ml buffer). The procedure was repeated with the second half of the protein containing supernatant. The protein containing elutions were pooled and dialyzed in PBS, pH 8.0 containing 1 M urea at 4°C over night. The dialysis buffer was exchanged with 0.5 M urea in PBS (pH 8.0) and dialysis was continued for additional 4 hours. Subsequently, the buffer was exchanged with PBS (pH 8.0) without urea and dialyzed for additional 4 hours. The protein concentration was determined with the Pierce BCA Protein Assay Kit (ThermoFisher) according to the instructions of the manufacturer. The protein was stored at -80°C until usage.

### EXAMPLE 7: ISOLATION OF APXA TOXINS FROM APP

Solution APPVX contains 1 g D(+)-Glucose, monohydrate, 0.1 g L-glutamine, 0.362 g L-cysteine hydrochloride monohydrate and 10 mL nuclease-free H₂O. APP isolates were plated onto chocolate agar comprising IsovitaleX and incubated at 37°C over night. Three APP colonies were combined and used to inoculate 3 ml columbia medium (10 mM Ca²⁺, 0.2 mg/mL NAD, 1 % APPVX) and the obtained culture was incubated at 37°C under shaking for 5 hours. Subsequently, the culture was centrifuged at 3200 g and 4°C for 20 minutes. The clear supernatant was transferred into a new tube and 44.05 g ammonia sulfate were gradually added and mixed until the ammonia sulfate completely dissolved. Subsequently, the solution was incubated at 4°C over night. Precipitated proteins were centrifuged at 3200 g and 4°C for 3 hours and the supernatant was removed. The protein pellet was air dried and subsequently suspended in 1 mL PBS comprising 6 M urea. To remove residual buffer, the obtained protein solution was purified via gel filtration on PDMidiTrap G25 columns (GE Healthcare). After equilibration with PBS comprising 6 M urea 1 ml of the protein solution was loaded onto the gel filtration column and subsequently eluted with 1.5 ml PBS comprising 6 M urea. Subsequently, the eluted proteins were stored at -20°C. Identity of the eluted proteins was confirmed via SDS-PAGE.

### EXAMPLE 8: ESTABLISHMENT OF A CYTOTOXICITY ASSAY

To analyze the cytotoxic activity of recombinant ApxA proteins (wild type and toxoid forms), a cytotoxicity assay using BL3 cells (BL3.1 CRL-2306) as target cells. BL3 cells were obtained from bovine B-cells and it is known that they can be lysed by ApxA toxins (Tu, A. H.; Hausler, C.; Young, R.; Struck, D. K. (1994): Differential expression of the cytotoxic and hemolytic activities of the ApxIIA toxin from Actinobacillus pleuropneumoniae. In Infection and immunity 62 (5), pp. 2119-2121). ApxA toxins as obtained in Example 7 were used as a positive control and PBS comprising 6 M urea served as negative control.

BL3-cells were cultivated in medium (DMEM containing 100 U/mL penicillin, 100 µg/mL streptomycin, 10 % FCS und 1x non-essential amino acids) at 37 °C and 5 % CO₂. T75 flasks with 25 ml of the medium were used for cultivation. BL3 cells were passaged weekly with 50,000 cells/cm² used to seed a new flask.

For utilization in the cytotoxicity assay 50 µl BL3 cells in medium were transferred into a well of 96 well plate. Recombinant proteins and controls were diluted in DMEM. 50 µl of the diluted recombinant proteins and controls were added to each well comprising the cells. Subsequently, the plate was incubated at 37°C and 5% CO₂ for 24 hours. After assessing the cell morphology microscopically 10 µl of WST-1 (Sigma Aldrich) was added to each well and the plate was incubated at 37°C and 5% CO₂ for a further 24 hours.

Subsequently, absorption is determined with an ELISA reader at 450 nm. WST-1 is cleaved by a complex cellular mechanism on the cell surface of viable cells to a soluble formazan. Accordingly, the amount of formed formazan dye and the extinction read in the ELISA reader directly correlate with the number of metabolically active cells in culture.

### EXAMPLE 9: ESTABLISHMENT OF A HEMOLYTIC ASSAY

To analyze the hemolytic activity of recombinant ApxA proteins (wild type and toxoid forms), a hemolytic assay was established. Cell culture plates comprising Columbia agar with 5% sheep blood (Bection Dickinson) were used. Holes with a diameter of 3 mm were punched out of the cell culture plates. 10 µl of the respective recombinant protein and control samples were filled into the hole and the plates was incubated at 37°C over night. ApxA toxins as obtained in Example 7 were used as a positive control and PBS comprising 6 M urea served as negative control.

Hemolytic activity is considered to present when the sheep blood agar decolorizes and thus appears whitish.

### EXAMPLE 10: ISOLATION OF APXA TOXINS FROM APP

APP strains ST2, ST5, ST7, and ST8 were used to isolate supernatants comprising ApxA toxins using the expression and isolation method described in example 7. SDS-PAGE revealed that the supernatants contained bands at the expected molecular weight positions corresponding to ApxIA, ApxIIA, ApxIIIA, and ApxIVA (data not shown). Thus, each of the obtained supernatants of respective strains ST2, ST5, ST7, and ST8 comprises a mixture of ApxIA, ApxIIA, ApxIIIA, and ApxIVA.

### EXAMPLE 11: CYTOTOXICITY OF APXA FROM APP IN BL-3 CELLS

Using the method of example 8 50 µl of the ApxA toxins of example 7 obtained from APP (APP strains ST2, ST5, ST7, and ST8) were tested in increasing dilutions in DMEM (1:8, 1:16, 1:32; 1:64, 1:128; 1:256, 1:512; 1:1024, 1:2048, 1:4096).

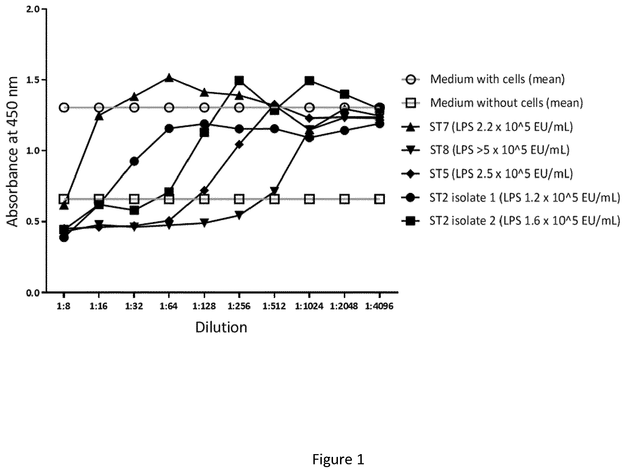

A cytotoxic effect of the APP supernatants was observed that decreased with increasing dilutions (see figure 1). Isolated toxins of ST8 exhibited the highest cytotoxicity whereas isolated toxins of ST7 showed the lowest toxicity. No correlation of LPS in the ApxA preparation on cytotoxicity could be observed (data not shown).

### EXAMPLE 12: CYTOTOXICITY OF RECOMBINANT FORMS OF APXIA AND APXIIIA IN BL-3 CELLS

Using the method of example 8 the following samples were tested

**Table 4**

| Designation | Content |
|---|---|
| ApxIA wildtype | 844 µg/ml purified protein obtained from *E. coli* comprising pACYC184 and pQE-80L comprising coding sequence for wild type ApxIA (SEQ ID NO: 7) and ApxIC (SEQ ID NO: 13) according to example 6 in PBS |
| ApxIA toxoid form | 722 µg/ml purified protein obtained from *E. coli* comprising pACYC184 and pQE-80L comprising coding sequence for toxoid ApxIA K560A and K686A (SEQ ID NO: 10) and ApxIC (SEQ ID NO: 13) according to example 6 in PBS |
| ApxIIIA wildtype | 591 µg/ml purified protein obtained from *E. coli* comprising pACYC184 and pQE-80L comprising coding sequence for wild type ApxIIIA (SEQ ID NO: 9) and ApxIIIC (SEQ ID NO: 15) according to example 6 in PBS |
| ApxIIIA toxoid form | 1033 µg/ml purified protein obtained from *E. coli* comprising pACYC184 and pQE-80L comprising coding sequence for toxoid ApxIIIA K560A and K686A (SEQ ID NO: 12) and ApxIIIC (SEQ ID NO: 15) according to example 6 in PBS |
| ST2 | Isolated supernatant from APP ST2 according to example 7 in PBS 6 M urea (positive control) |
| Medium without cells in well | DMEM (negative control) |
| PBS control | PBS and cells |

50 µl of each sample was tested in increasing dilutions in DMEM (1:2, 1:4, 1:8, 1:16, 1:32; 1:64, 1:128; 1:256, 1:512).

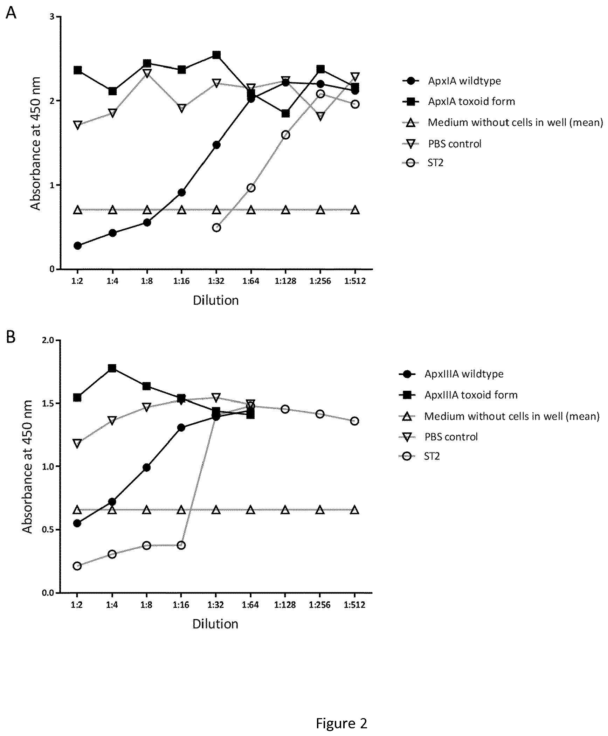

The control with cells used dilutions of PBS to evaluate an influence of PBS. No effect was found (see figure 2). The results are illustrated in figure 2.

As illustrated in figure 2A the cytotoxicity assay demonstrated that recombinant wild type ApxIA lysed BL3 cells and thus was active. Starting with a dilution of 1:64 (13.1 µg/ml wildtype ApxIA toxin) the values for the viability of the cells were comparable to those to the PBS control. The viability of BL3 cells that had been incubated with the ApxIA toxoid form were comparable to that of the PBS control. This also applied to the cell morphology (data not shown).

Since the toxoid form of ApxIA had the same effect on cell viability and morphology as the PBS control while the wildtype ApxIA form had a strong cytotoxic effect on BL3 cells it can be concluded that the toxoid form of ApxIA has no cytotoxic effect. Similar results were obtained with regard to the toxoid forms of ApxIIA (data not shown) and ApxIIIA (figure 2 B).

### EXAMPLE 13: HEMOLYTIC ACTIVITY OF APXA FROM APP

Using the method of example 9 10 µl of the ApxA toxins of example 7 obtained from APP (APP strains ST2, ST5, and ST8) were tested.

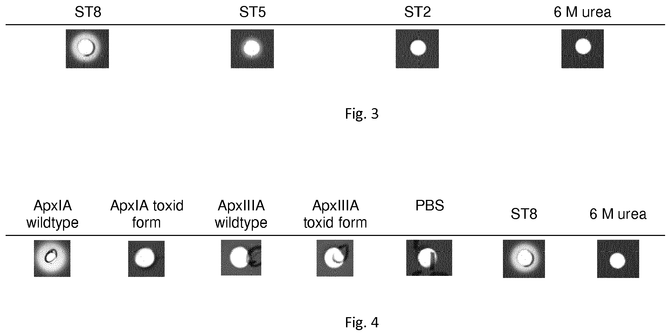

The results are illustrated in figure 3. Strain ST8 exhibited a strong hemolysis, ST5 a moderate hemolysis and ST2 no hemolysis as can be inferred from the decreasing whitish color from left to right in figure 3. It is known that not each of ApxIA, ApxIIA and ApxIIA is capable of lysing erythrocytes. Since different strains secret different amounts of the respective ApxA forms the supernatants from the respective strains which comprise mixtures of the different ApxA forms can exhibit different hemolytic activity.

In sum, it can be demonstrated that hemolytic activity of ApxA toxins can be demonstrated in a method involving the use of Columbia agar comprising 5% sheep blood as described in example 9.

### EXAMPLE 14: HEMOLYTIC ACTIVITY OF RECOMBINANT FORMS OF APXIA

Using the method of example 9 the following samples were tested

**Table 5**

| Designation | Content |
|---|---|
| ApxIA wildtype | 844 µg/ml purified protein obtained from E. coli comprising pACYC184 and pQE-80L comprising coding sequence for wild type ApxIA (SEQ ID NO: 7) and ApxIC (SEQ ID NO: 13) according to example 6 in PBS |
| ApxIA toxoid form | 722 µg/ml purified protein obtained from E. coli comprising pACYC184 and pQE-80L comprising coding sequence for toxoid ApxIA K560A and K686A (SEQ ID NO: 10) and ApxIC (SEQ ID NO: 13) according to example 6 in PBS |
| ApxIIIA wildtype | 591 µg/ml purified protein obtained from *E. coli* comprising pACYC184 and pQE-80L comprising coding sequence for wild type ApxIIIA (SEQ ID NO: 9) and ApxIIIC (SEQ ID NO: 15) according to example 6 in PBS |
| ApxIIIA toxoid form | 1033 µg/ml purified protein obtained from *E. coli* comprising pACYC184 and pQE-80L comprising coding sequence for toxoid ApxIIIA K560A and K686A (SEQ ID NO: 12) and ApxIIIC (SEQ ID NO: 15) according to example 6 in PBS |
| ST8 | Isolated supernatant from APP ST8 according to example 7 in PBS 6 M urea (positive control) |
| 6 M urea | PBS 6 M urea (negative control) |
| PBS | PBS (negative control) |

The results are illustrated in figure 4. While the ApxIA wildtype form lyses erythrocytes the ApxIA toxoid form does not lyse erythrocytes. ApxIIIA wildtype form showed no hemolytic activity as it is also described in literature.

Since the toxoid form of ApxIA had the same, i.e. absent, hemolytic effect as the PBS control while the wildtype ApxIA form had a strong hemolytic effect on sheep erythrocytes it can be concluded that the toxoid for of ApxIA has no hemolytic effect. Similar results were obtained with regard to the toxoid forms of ApxIIA (data not shown).

## Claims

1. ApxIA, ApxIIA or ApxIIIA polypeptide of *Actinobacillus pleuropneumoniae* wherein
a. the wild type amino acid sequence of
i. ApxIA as set forth SEQ ID NO:1 is modified in at least one amino acid selected from the group consisting of K560 and K686,
ii. ApxIIA as set forth SEQ ID NO:2 is modified in at least one amino acid selected from the group consisting of K557 and N687, and
iii. ApxIIIA as set forth SEQ ID NO: 3 is modified in at least one amino acid selected from the group consisting of K571 and K702,
and the at least one amino acid is exchanged by an amino acid not susceptible to acylation; or
b. an analogue or a fragment of the modified amino acid sequence as defined under a. which is at least 90% homologous to the modified amino acid sequence as defined under a. and a fragment comprises at least 50% of the consecutive amino acids of the modified amino acid sequence as defined under a., and the analogue or fragment comprises the amino acid not susceptible to acylation as defined under a; or
c. the wild type amino acid sequence of
i. ApxIA as set forth SEQ ID NO:1 containing deletions comprising at least one amino acid selected from the group consisting of K560 and K686,
ii. ApxIIA as set forth SEQ ID NO:2 containing deletions comprising at least one amino acid selected from the group consisting of K557 and N687, and
iii. ApxIIIA as set forth SEQ ID NO:3 containing deletions comprising at least one amino acid selected from the group consisting of K571 and K702,
wherein the deletions do not delete more than 50% of the wild type amino acid sequence;
d. an analogue of the amino acid sequence comprising deletions as defined under c. which is at least 90% homologous to the amino acid sequence comprising deletions as defined under c.

2. ApxIA, ApxIIA or ApxIIIA polypeptide of claim 1 wherein the amino acid not susceptible to acylation is independently selected from the group consisting of alanine, glycine, isoleucine, leucine, methionine, valine, serine, threonine, asparagine, glutamine, aspartic acid, histidine, aspartic acid, cysteine, proline, phenylalanine, tyrosine, tryptophan and glutamic acid; preferably selected from the group consisting of alanine, glycine, serine, threonine, valine, isoleucine and leucine;
most preferably selected from the group consisting of alanine, glycine, serine.

3. ApxIA, ApxIIA or ApxIIIA polypeptide of any preceding claim, wherein the wild type amino acid sequence of
a. ApxIA as set forth SEQ ID NO:1 is modified at K560 and K686,
b. ApxIIA as set forth SEQ ID NO:2 is modified at K557 and N687, and
c. ApxIIIA as set forth SEQ ID NO: 3 is modified at K571 and K702.

4. ApxIA, ApxIIA or ApxIIIA polypeptide of any preceding claim, wherein
a. the modified ApxIA wild type amino acid sequence is SEQ ID NO:4,
b. the modified ApxIIA wild type amino acid sequence is SEQ ID NO:5, and
c. the modified ApxIIIA wild type amino acid sequence is SEQ ID NO:6.

5. A nucleic acid molecule comprising a sequence of nucleotides that encodes an ApxIA, ApxIIA or ApxIIIA polypeptide according to any one of claims 1 to 4.

6. A vector comprising the nucleic acid molecule of claim 5.

7. A microorganism comprising the nucleic acid molecule according to any one of claims 5 or 6.

8. The microorganism of claim 7 wherein the microorganism is an *Escherichia coli* strain or an *Actinobacillus strain, preferably on Actinobacillus pleuropneumoniae* strain.

9. Vaccine composition comprising
a. at least one ApxIA, ApxIIA or ApxIIIA polypeptide according to any one of claims 1 to 4,
b. the nucleic acid molecule according to claim 5 or the vector according claim 6, or
c. a microorganism according to any one of claims 7 or 8;
and at least a pharmaceutical carrier, a diluent, or/and an adjuvant.

10. Vaccine composition of claim 9, wherein the vaccine composition of claim 9.a. is a subunit vaccine.

11. Vaccine composition of claim 10 wherein the subunit vaccine comprises at least one further polypeptide of *Actinobacillus pleuropneumoniae.*

12. Vaccine composition of claim 9, wherein the microorganism of claim 9.c. is a live vaccine, preferably an *Actinobacillus pleuropneumoniae* strain.

13. Vaccine composition of claim 12 comprising at least two different *Actinobacillus pleuropneumoniae* strains.

14. Vaccine composition of any one of claims 12 or 13 wherein the apxIV gene or/and the sxy gene is deleted.

15. Vaccine composition of any preceding claim for use in the prophylactic, metaphylactic or therapeutic treatment of a pneumonia, a pleurisy or a pleuropneumonia, in particular, of a pneumonia, a pleurisy or a pleuropneumonia caused by *Actinobacillus pleuropneumoniae,* wherein optionally the vaccine composition is to be administered intramuscularly, intradermally, intravenously, subcutaneously, or by mucosal application.


SEQUENCE LISTING

<110> IDT Biologika GmbH

<120> Inactivated ApxIA, ApxIIA and ApxIIIA toxins

<130> P46559-EP

<160> 34

<170> BiSSAP 1.3.6

<210> 1
<211> 1022
<212> PRT
<213> Actonbacillus pleuropneumoniae

<220>
<223> ApxIA wild type

<400> 1
Met Ala Asn Ser Gln Leu Asp Arg Val Lys Gly Leu Ile Asp Ser Leu
1               5                   10                  15
Asn Gln His Thr Lys Ser Ala Ala Lys Ser Gly Ala Gly Ala Leu Lys
            20                  25                  30
Asn Gly Leu Gly Gln Val Lys Gln Ala Gly Gln Lys Leu Ile Leu Tyr
        35                  40                  45
Ile Pro Lys Asp Tyr Gln Ala Ser Thr Gly Ser Ser Leu Asn Asp Leu
    50                  55                  60
Val Lys Ala Ala Glu Ala Leu Gly Ile Glu Val His Arg Ser Glu Lys
65                  70                  75                  80
Asn Gly Thr Ala Leu Ala Lys Glu Leu Phe Gly Thr Thr Glu Lys Leu
                85                  90                  95
Leu Gly Phe Ser Glu Arg Gly Ile Ala Leu Phe Ala Pro Gln Phe Asp
            100                 105                 110
Lys Leu Leu Asn Lys Asn Gln Lys Leu Ser Lys Ser Leu Gly Gly Ser
        115                 120                 125
Ser Glu Ala Leu Gly Gln Arg Leu Asn Lys Thr Gln Thr Ala Leu Ser
    130                 135                 140
Ala Leu Gln Ser Phe Leu Gly Thr Ala Ile Ala Gly Met Asp Leu Asp
145                 150                 155                 160
Ser Leu Leu Arg Arg Arg Arg Asn Gly Glu Asp Val Ser Gly Ser Glu
                165                 170                 175
Leu Ala Lys Ala Gly Val Asp Leu Ala Ala Gln Leu Val Asp Asn Ile
            180                 185                 190
Ala Ser Ala Thr Gly Thr Val Asp Ala Phe Ala Glu Gln Leu Gly Lys
        195                 200                 205
Leu Gly Asn Ala Leu Ser Asn Thr Arg Leu Ser Gly Leu Ala Ser Lys
    210                 215                 220
Leu Asn Asn Leu Pro Asp Leu Ser Leu Ala Gly Pro Gly Phe Asp Ala
225                 230                 235                 240
Val Ser Gly Ile Leu Ser Val Val Ser Ala Ser Phe Ile Leu Ser Asn
                245                 250                 255
Lys Asp Ala Asp Ala Gly Thr Lys Ala Ala Ala Gly Ile Glu Ile Ser
            260                 265                 270
Thr Lys Ile Leu Gly Asn Ile Gly Lys Ala Val Ser Gln Tyr Ile Ile
        275                 280                 285
Ala Gln Arg Val Ala Ala Gly Leu Ser Thr Thr Ala Ala Thr Gly Gly
    290                 295                 300
Leu Ile Gly Ser Val Val Ala Leu Ala Ile Ser Pro Leu Ser Phe Leu
305                 310                 315                 320
Asn Val Ala Asp Lys Phe Glu Arg Ala Lys Gln Leu Glu Gln Tyr Ser

325                 330                 335
Glu Arg Phe Lys Lys Phe Gly Tyr Glu Gly Asp Ser Leu Leu Ala Ser
        340                 345                 350
Phe Tyr Arg Glu Thr Gly Ala Ile Glu Ala Ala Leu Thr Thr Ile Asn
        355                 360                 365
Ser Val Leu Ser Ala Ala Ser Ala Gly Val Gly Ala Ala Ala Thr Gly
    370                 375                 380
Ser Leu Val Gly Ala Pro Val Ala Ala Leu Val Ser Ala Ile Thr Gly
385                 390                 395                 400
Ile Ile Ser Gly Ile Leu Asp Ala Ser Lys Gln Ala Ile Phe Glu Arg
                405                 410                 415
Val Ala Thr Lys Leu Ala Asn Lys Ile Asp Glu Trp Glu Lys Lys His
            420                 425                 430
Gly Lys Asn Tyr Phe Glu Asn Gly Tyr Asp Ala Arg His Ser Ala Phe
        435                 440                 445
Leu Glu Asp Thr Phe Glu Leu Leu Ser Gln Tyr Asn Lys Glu Tyr Ser
    450                 455                 460
Val Glu Arg Val Val Ala Ile Thr Gln Gln Arg Trp Asp Val Asn Ile
465                 470                 475                 480
Gly Glu Leu Ala Gly Ile Thr Arg Lys Gly Ala Asp Ala Lys Ser Gly
                485                 490                 495
Lys Ala Tyr Val Asp Phe Phe Glu Glu Gly Lys Leu Leu Glu Lys Asp
            500                 505                 510
Pro Asp Arg Phe Asp Lys Lys Val Phe Asp Pro Leu Glu Gly Lys Ile
        515                 520                 525
Asp Leu Ser Ser Ile Asn Lys Thr Thr Leu Leu Lys Phe Ile Thr Pro
    530                 535                 540
Val Phe Thr Ala Gly Glu Glu Ile Arg Glu Arg Lys Gln Thr Gly Lys
545                 550                 555                 560
Tyr Glu Tyr Met Thr Glu Leu Phe Val Lys Gly Lys Glu Lys Trp Val
                565                 570                 575
Val Thr Gly Val Gln Ser His Asn Ala Ile Tyr Asp Tyr Thr Asn Leu
            580                 585                 590
Ile Gln Leu Ala Ile Asp Lys Lys Gly Glu Lys Arg Gln Val Thr Ile
        595                 600                 605
Glu Ser His Leu Gly Glu Lys Asn Asp Arg Ile Tyr Leu Ser Ser Gly
    610                 615                 620
Ser Ser Ile Val Tyr Ala Gly Asn Gly His Asp Val Ala Tyr Tyr Asp
625                 630                 635                 640
Lys Thr Asp Thr Gly Tyr Leu Thr Phe Asp Gly Gln Ser Ala Gln Lys
                645                 650                 655
Ala Gly Glu Tyr Ile Val Thr Lys Glu Leu Lys Ala Asp Val Lys Val
            660                 665                 670
Leu Lys Glu Val Val Lys Thr Gln Asp Ile Ser Val Gly Lys Arg Ser
        675                 680                 685
Glu Lys Leu Glu Tyr Arg Asp Tyr Glu Leu Ser Pro Phe Glu Leu Gly
    690                 695                 700
Asn Gly Ile Arg Ala Lys Asp Glu Leu His Ser Val Glu Glu Ile Ile
705                 710                 715                 720
Gly Ser Asn Arg Lys Asp Lys Phe Phe Gly Ser Arg Phe Thr Asp Ile
                725                 730                 735
Phe His Gly Ala Lys Gly Asp Asp Glu Ile Tyr Gly Asn Asp Gly His
            740                 745                 750
Asp Ile Leu Tyr Gly Asp Asp Gly Asn Asp Val Ile His Gly Gly Asp
        755                 760                 765
Gly Asn Asp His Leu Val Gly Gly Asn Gly Asn Asp Arg Leu Ile Gly
    770                 775                 780
Gly Lys Gly Asn Asn Phe Leu Asn Gly Gly Asp Gly Asp Asp Glu Leu
785                 790                 795                 800
Gln Val Phe Glu Gly Gln Tyr Asn Val Leu Leu Gly Gly Ala Gly Asn
                805                 810                 815
Asp Ile Leu Tyr Gly Ser Asp Gly Thr Asn Leu Phe Asp Gly Gly Val
            820                 825                 830

Gly Asn Asp Lys Ile Tyr Gly Gly Leu Gly Lys Asp Ile Tyr Arg Tyr
        835                 840                 845
Ser Lys Glu Tyr Gly Arg His Ile Ile Ile Glu Lys Gly Gly Asp Asp
    850                 855                 860
Asp Thr Leu Leu Leu Ser Asp Leu Ser Phe Lys Asp Val Gly Phe Ile
865                 870                 875                 880
Arg Ile Gly Asp Asp Leu Leu Val Asn Lys Arg Ile Gly Gly Thr Leu
                885                 890                 895
Tyr Tyr His Glu Asp Tyr Asn Gly Asn Ala Leu Thr Ile Lys Asp Trp
            900                 905                 910
Phe Lys Glu Gly Lys Glu Gly Gln Asn Asn Lys Ile Glu Lys Ile Val
        915                 920                 925
Asp Lys Asp Gly Ala Tyr Val Leu Ser Gln Tyr Leu Thr Glu Leu Thr
    930                 935                 940
Ala Pro Gly Arg Gly Ile Asn Tyr Phe Asn Gly Leu Glu Glu Lys Leu
945                 950                 955                 960
Tyr Tyr Gly Glu Gly Tyr Asn Ala Leu Pro Gln Leu Arg Lys Asp Ile
                965                 970                 975
Glu Gln Ile Ile Ser Ser Thr Gly Ala Leu Thr Gly Glu His Gly Gln
            980                 985                 990
Val Leu Val Gly Ala Gly Gly Pro Leu Ala Tyr Ser Asn Ser Pro Asn
        995                 1000                1005
Ser Ile Pro Asn Ala Phe Ser Asn Tyr Leu Thr Gln Ser Ala
    1010                1015                1020

<210> 2
<211> 956
<212> PRT
<213> Actonbacillus pleuropneumoniae


<220>
<223> ApxIIA wild type

<400> 2
Met Ser Lys Ile Thr Leu Ser Ser Leu Lys Ser Ser Leu Gln Gln Gly
1               5                   10                  15
Leu Lys Asn Gly Lys Asn Lys Leu Asn Gln Ala Gly Thr Thr Leu Lys
            20                  25                  30
Asn Gly Leu Thr Gln Thr Gly His Ser Leu Gln Asn Gly Ala Lys Lys
        35                  40                  45
Leu Ile Leu Tyr Ile Pro Gln Gly Tyr Asp Ser Gly Gln Gly Asn Gly
    50                  55                  60
Val Gln Asp Leu Val Lys Ala Ala Asn Asp Leu Gly Ile Glu Val Trp
65                  70                  75                  80
Arg Glu Glu Arg Ser Asn Leu Asp Ile Ala Lys Thr Ser Phe Asp Thr
                85                  90                  95
Thr Gln Lys Ile Leu Gly Phe Thr Asp Arg Gly Ile Val Leu Phe Ala
            100                 105                 110
Pro Gln Leu Asp Asn Leu Leu Lys Lys Asn Pro Lys Ile Gly Asn Thr
        115                 120                 125
Leu Gly Ser Ala Ser Ser Ile Ser Gln Asn Ile Gly Lys Ala Asn Thr
    130                 135                 140
Val Leu Gly Gly Ile Gln Ser Ile Leu Gly Ser Val Leu Ser Gly Val
145                 150                 155                 160
Asn Leu Asn Glu Leu Leu Gln Asn Lys Asp Pro Asn Gln Leu Glu Leu
                165                 170                 175
Ala Lys Ala Gly Leu Glu Leu Thr Asn Glu Leu Val Gly Asn Ile Ala
            180                 185                 190
Ser Ser Val Gln Thr Val Asp Ala Phe Ala Glu Gln Ile Ser Lys Leu
        195                 200                 205
Gly Ser His Leu Gln Asn Val Lys Gly Leu Gly Gly Leu Ser Asn Lys
    210                 215                 220

Leu Gln Asn Leu Pro Asp Leu Gly Lys Ala Ser Leu Gly Leu Asp Ile
225                 230                 235                 240
Ile Ser Gly Leu Leu Ser Gly Ala Ser Ala Gly Leu Ile Leu Ala Asp
                245                 250                 255
Lys Glu Ala Ser Thr Glu Lys Lys Ala Ala Ala Gly Val Glu Phe Ala
            260                 265                 270
Asn Gln Ile Ile Gly Asn Val Thr Lys Ala Val Ser Ser Tyr Ile Leu
        275                 280                 285
Ala Gln Arg Val Ala Ser Gly Leu Ser Ser Thr Gly Pro Val Ala Ala
    290                 295                 300
Leu Ile Ala Ser Thr Val Ala Leu Ala Val Ser Pro Leu Ser Phe Leu
305                 310                 315                 320
Asn Val Ala Asp Lys Phe Lys Gln Ala Asp Leu Ile Lys Ser Tyr Ser
                325                 330                 335
Glu Arg Phe Gln Lys Leu Gly Tyr Asp Gly Asp Arg Leu Leu Ala Asp
            340                 345                 350
Phe His Arg Glu Thr Gly Thr Ile Asp Ala Ser Val Thr Thr Ile Asn
        355                 360                 365
Thr Ala Leu Ala Ala Ile Ser Gly Gly Val Gly Ala Ala Ser Ala Gly
    370                 375                 380
Ser Leu Val Gly Ala Pro Val Ala Leu Leu Val Ala Gly Val Thr Gly
385                 390                 395                 400
Leu Ile Thr Thr Ile Leu Glu Tyr Ser Lys Gln Ala Met Phe Glu His
                405                 410                 415
Val Ala Asn Lys Val His Asp Arg Ile Val Glu Trp Glu Lys Lys His
            420                 425                 430
Asn Lys Asn Tyr Phe Glu Gln Gly Tyr Asp Ser Arg His Leu Ala Asp
        435                 440                 445
Leu Gln Asp Asn Met Lys Phe Leu Ile Asn Leu Asn Lys Glu Leu Gln
    450                 455                 460
Ala Glu Arg Val Val Ala Ile Thr Gln Gln Arg Trp Asp Asn Gln Ile
465                 470                 475                 480
Gly Asp Leu Ala Ala Ile Ser Arg Arg Thr Asp Lys Ile Ser Ser Gly
                485                 490                 495
Lys Ala Tyr Val Asp Ala Phe Glu Glu Gly Gln His Gln Ser Tyr Asp
            500                 505                 510
Ser Ser Val Gln Leu Asp Asn Lys Asn Gly Ile Ile Asn Ile Ser Asn
        515                 520                 525
Thr Asn Arg Lys Thr Gln Ser Val Leu Phe Arg Thr Pro Leu Leu Thr
    530                 535                 540
Pro Gly Glu Glu Asn Arg Glu Arg Ile Gln Glu Gly Lys Asn Ser Tyr
545                 550                 555                 560
Ile Thr Lys Leu His Ile Gln Arg Val Asp Ser Trp Thr Val Thr Asp
                565                 570                 575
Gly Asp Ala Ser Ser Ser Val Asp Phe Thr Asn Val Val Gln Arg Ile
            580                 585                 590
Ala Val Lys Phe Asp Asp Ala Gly Asn Ile Ile Glu Ser Lys Asp Thr
        595                 600                 605
Lys Ile Ile Ala Asn Leu Gly Ala Gly Asn Asp Asn Val Phe Val Gly
    610                 615                 620
Ser Ser Thr Thr Val Ile Asp Gly Gly Asp Gly His Asp Arg Val His
625                 630                 635                 640
Tyr Ser Arg Gly Glu Tyr Gly Ala Leu Val Ile Asp Ala Thr Ala Glu
                645                 650                 655
Thr Glu Lys Gly Ser Tyr Ser Val Lys Arg Tyr Val Gly Asp Ser Lys
            660                 665                 670
Ala Leu His Glu Thr Ile Ala Thr His Gln Thr Asn Val Gly Asn Arg
        675                 680                 685
Glu Glu Lys Ile Glu Tyr Arg Arg Glu Asp Asp Arg Phe His Thr Gly
    690                 695                 700
Tyr Thr Val Thr Asp Ser Leu Lys Ser Val Glu Glu Ile Ile Gly Ser
705                 710                 715                 720
Gln Phe Asn Asp Ile Phe Lys Gly Ser Gln Phe Asp Asp Val Phe His

725                 730                 735
Gly Gly Asn Gly Val Asp Thr Ile Asp Gly Asn Asp Gly Asp Asp His
            740                 745                 750
Leu Phe Gly Gly Ala Gly Asp Asp Val Ile Asp Gly Gly Asn Gly Asn
        755                 760                 765
Asn Phe Leu Val Gly Gly Thr Gly Asn Asp Ile Ile Ser Gly Gly Lys
    770                 775                 780
Asp Asn Asp Ile Tyr Val His Lys Thr Gly Asp Gly Asn Asp Ser Ile
785                 790                 795                 800
Thr Asp Ser Gly Gly Gln Asp Lys Leu Ala Phe Ser Asp Val Asn Leu
                805                 810                 815
Lys Asp Leu Thr Phe Lys Lys Val Asp Ser Ser Leu Glu Ile Ile Asn
            820                 825                 830
Gln Lys Gly Glu Lys Val Arg Ile Gly Asn Trp Phe Leu Glu Asp Asp
        835                 840                 845
Leu Ala Ser Thr Val Ala Asn Tyr Lys Ala Thr Asn Asp Arg Lys Ile
    850                 855                 860
Glu Glu Ile Ile Gly Lys Gly Gly Glu Arg Ile Thr Ser Glu Gln Val
865                 870                 875                 880
Asp Lys Leu Ile Lys Glu Gly Asn Asn Gln Ile Ser Ala Glu Ala Leu
                885                 890                 895
Ser Lys Val Val Asn Asp Tyr Asn Thr Ser Lys Asp Arg Gln Asn Val
            900                 905                 910
Ser Asn Ser Leu Ala Lys Leu Ile Ser Ser Val Gly Ser Phe Thr Ser
        915                 920                 925
Ser Ser Asp Phe Arg Asn Asn Leu Gly Thr Tyr Val Pro Ser Ser Ile
    930                 935                 940
Asp Val Ser Asn Asn Ile Gln Leu Ala Arg Ala Ala
945                 950                 955

<210> 3
<211> 1052
<212> PRT
<213> Actonbacillus pleuropneumoniae

<220>
<223> ApxIIIA wild type

<400> 3
Met Ser Thr Trp Ser Ser Met Leu Ala Asp Leu Lys Lys Arg Ala Glu
1               5                   10                  15
Glu Ala Lys Arg Gln Ala Lys Lys Gly Tyr Asp Val Thr Lys Asn Gly
            20                  25                  30
Leu Gln Tyr Gly Val Ser Gln Ala Lys Leu Gln Ala Leu Ala Ala Gly
        35                  40                  45
Lys Ala Val Gln Lys Tyr Gly Asn Lys Leu Val Leu Val Ile Pro Lys
    50                  55                  60
Glu Tyr Asp Gly Ser Val Gly Asn Gly Phe Phe Asp Leu Val Lys Ala
65                  70                  75                  80
Ala Glu Glu Leu Gly Ile Gln Val Lys Tyr Val Asn Arg Asn Glu Leu
                85                  90                  95
Glu Val Ala His Lys Ser Leu Gly Thr Ala Asp Gln Phe Leu Gly Leu
            100                 105                 110
Thr Glu Arg Gly Leu Thr Leu Phe Ala Pro Gln Leu Asp Gln Phe Leu
        115                 120                 125
Gln Lys His Ser Lys Ile Ser Asn Val Val Gly Ser Ser Thr Gly Asp
    130                 135                 140
Ala Val Ser Lys Leu Ala Lys Ser Gln Thr Ile Ile Ser Gly Ile Gln
145                 150                 155                 160
Ser Val Leu Gly Thr Val Leu Ala Gly Ile Asn Leu Asn Glu Ala Ile
                165                 170                 175
Ile Ser Gly Gly Ser Glu Leu Glu Leu Ala Glu Ala Gly Val Ser Leu

            180                 185                 190
Ala Ser Glu Leu Val Ser Asn Ile Ala Lys Gly Thr Thr Thr Ile Asp
        195                 200                 205
Ala Phe Thr Thr Gln Ile Gln Asn Phe Gly Lys Leu Val Glu Asn Ala
    210                 215                 220
Lys Gly Leu Gly Gly Val Gly Arg Gln Leu Gln Asn Ile Ser Gly Ser
225                 230                 235                 240
Ala Leu Ser Lys Thr Gly Leu Gly Leu Asp Ile Ile Ser Ser Leu Leu
                245                 250                 255
Ser Gly Val Thr Ala Ser Phe Ala Leu Ala Asn Lys Asn Ala Ser Thr
            260                 265                 270
Ser Thr Lys Val Ala Ala Gly Phe Glu Leu Ser Asn Gln Val Ile Gly
        275                 280                 285
Gly Ile Thr Lys Ala Val Ser Ser Tyr Ile Leu Ala Gln Arg Leu Ala
    290                 295                 300
Ala Gly Leu Ser Thr Thr Gly Pro Ala Ala Ala Leu Ile Ala Ser Ser
305                 310                 315                 320
Ile Ser Leu Ala Ile Ser Pro Leu Ala Phe Leu Arg Val Ala Asp Asn
                325                 330                 335
Phe Asn Arg Ser Lys Glu Ile Gly Glu Phe Ala Glu Arg Phe Lys Lys
            340                 345                 350
Leu Gly Tyr Asp Gly Asp Lys Leu Leu Ser Glu Phe Tyr His Glu Ala
        355                 360                 365
Gly Thr Ile Asp Ala Ser Ile Thr Thr Ile Ser Thr Ala Leu Ser Ala
    370                 375                 380
Ile Ala Ala Gly Thr Ala Ala Ala Ser Ala Gly Ala Leu Val Gly Ala
385                 390                 395                 400
Pro Ile Thr Leu Leu Val Thr Gly Ile Thr Gly Leu Ile Ser Gly Ile
                405                 410                 415
Leu Glu Phe Ser Lys Gln Pro Met Leu Asp His Val Ala Ser Lys Ile
            420                 425                 430
Gly Asn Lys Ile Asp Glu Trp Glu Lys Lys Tyr Gly Lys Asn Tyr Phe
        435                 440                 445
Glu Asn Gly Tyr Asp Ala Arg His Lys Ala Phe Leu Glu Asp Ser Phe
    450                 455                 460
Ser Leu Leu Ser Ser Phe Asn Lys Gln Tyr Glu Thr Glu Arg Ala Val
465                 470                 475                 480
Leu Ile Thr Gln Gln Arg Trp Asp Glu Tyr Ile Gly Glu Leu Ala Gly
                485                 490                 495
Ile Thr Gly Lys Gly Asp Lys Leu Ser Ser Gly Lys Ala Tyr Val Asp
            500                 505                 510
Tyr Phe Gln Glu Gly Lys Leu Leu Glu Lys Lys Pro Asp Asp Phe Ser
        515                 520                 525
Lys Val Val Phe Asp Pro Thr Lys Gly Glu Ile Asp Ile Ser Asn Ser
    530                 535                 540
Gln Thr Ser Thr Leu Leu Lys Phe Val Thr Pro Leu Leu Thr Pro Gly
545                 550                 555                 560
Thr Glu Ser Arg Glu Arg Thr Gln Thr Gly Lys Tyr Glu Tyr Ile Thr
                565                 570                 575
Lys Leu Val Val Lys Gly Lys Asp Lys Trp Val Val Asn Gly Val Lys
            580                 585                 590
Asp Lys Gly Ala Val Tyr Asp Tyr Thr Asn Leu Ile Gln His Ala His
        595                 600                 605
Ile Ser Ser Ser Val Ala Arg Gly Glu Glu Tyr Arg Glu Val Arg Leu
    610                 615                 620
Val Ser His Leu Gly Asn Gly Asn Asp Lys Val Phe Leu Ala Ala Gly
625                 630                 635                 640
Ser Ala Glu Ile His Ala Gly Glu Gly His Asp Val Val Tyr Tyr Asp
                645                 650                 655
Lys Thr Asp Thr Gly Leu Leu Val Ile Asp Gly Thr Lys Ala Thr Glu
            660                 665                 670
Gln Gly Arg Tyr Ser Val Thr Arg Glu Leu Ser Gly Ala Thr Lys Ile
        675                 680                 685

Leu Arg Glu Val Ile Lys Asn Gln Lys Ser Ala Val Gly Lys Arg Glu
    690                 695                 700
Glu Thr Leu Glu Tyr Arg Asp Tyr Glu Leu Thr Gln Ser Gly Asn Ser
705                 710                 715                 720
Asn Leu Lys Ala His Asp Glu Leu His Ser Val Glu Glu Ile Ile Gly
                725                 730                 735
Ser Asn Gln Arg Asp Glu Phe Lys Gly Ser Lys Phe Arg Asp Ile Phe
            740                 745                 750
His Gly Ala Asp Gly Asp Asp Leu Leu Asn Gly Asn Asp Gly Asp Asp
        755                 760                 765
Ile Leu Tyr Gly Asp Lys Gly Asn Asp Glu Leu Arg Gly Asp Asn Gly
    770                 775                 780
Asn Asp Gln Leu Tyr Gly Gly Glu Gly Asn Asp Lys Leu Leu Gly Gly
785                 790                 795                 800
Asn Gly Asn Asn Tyr Leu Ser Gly Gly Asp Gly Asn Asp Glu Leu Gln
                805                 810                 815
Val Leu Gly Asn Gly Phe Asn Val Leu Arg Gly Gly Lys Gly Asp Asp
            820                 825                 830
Lys Leu Tyr Gly Ser Ser Gly Ser Asp Leu Leu Asp Gly Gly Glu Gly
        835                 840                 845
Asn Asp Tyr Leu Glu Gly Gly Asp Gly Ser Asp Phe Tyr Val Tyr Arg
    850                 855                 860
Ser Thr Ser Gly Asn His Thr Ile Tyr Asp Gln Gly Lys Ser Ser Asp
865                 870                 875                 880
Leu Asp Lys Leu Tyr Leu Ser Asp Phe Ser Phe Asp Arg Leu Leu Val
                885                 890                 895
Glu Lys Val Asp Asp Asn Leu Val Leu Arg Ser Asn Glu Ser Ser His
            900                 905                 910
Asn Asn Gly Val Leu Thr Ile Lys Asp Trp Phe Lys Glu Gly Asn Lys
        915                 920                 925
Tyr Asn His Lys Ile Glu Gln Ile Val Asp Lys Asn Gly Arg Lys Leu
    930                 935                 940
Thr Ala Glu Asn Leu Gly Thr Tyr Phe Lys Asn Ala Pro Lys Ala Asp
945                 950                 955                 960
Asn Leu Leu Asn Tyr Ala Thr Lys Glu Asp Gln Asn Glu Ser Asn Leu
                965                 970                 975
Ser Ser Leu Lys Thr Glu Leu Ser Lys Ile Ile Thr Asn Ala Gly Asn
            980                 985                 990
Phe Gly Val Ala Lys Gln Gly Asn Thr Gly Ile Asn Thr Ala Ala Leu
        995                 1000                1005
Asn Asn Glu Val Asn Lys Ile Ile Ser Ser Ala Asn Thr Phe Ala Thr
    1010                1015                1020
Ser Gln Leu Gly Gly Ser Gly Met Gly Thr Leu Pro Ser Thr Asn Val
1025                1030                1035                1040
Asn Ser Met Met Leu Gly Asn Leu Ala Arg Ala Ala
                1045                1050

<210> 4
<211> 1022
<212> PRT
<213> Actonbacillus pleuropneumoniae

<220>
<223> ApxIA K560A K686A

<400> 4
Met Ala Asn Ser Gln Leu Asp Arg Val Lys Gly Leu Ile Asp Ser Leu
1               5                   10                  15
Asn Gln His Thr Lys Ser Ala Ala Lys Ser Gly Ala Gly Ala Leu Lys
            20                  25                  30
Asn Gly Leu Gly Gln Val Lys Gln Ala Gly Gln Lys Leu Ile Leu Tyr
        35                  40                  45

Ile Pro Lys Asp Tyr Gln Ala Ser Thr Gly Ser Ser Leu Asn Asp Leu
    50                  55                  60
Val Lys Ala Ala Glu Ala Leu Gly Ile Glu Val His Arg Ser Glu Lys
65                  70                  75                  80
Asn Gly Thr Ala Leu Ala Lys Glu Leu Phe Gly Thr Thr Glu Lys Leu
                85                  90                  95
Leu Gly Phe Ser Glu Arg Gly Ile Ala Leu Phe Ala Pro Gln Phe Asp
            100                 105                 110
Lys Leu Leu Asn Lys Asn Gln Lys Leu Ser Lys Ser Leu Gly Gly Ser
        115                 120                 125
Ser Glu Ala Leu Gly Gln Arg Leu Asn Lys Thr Gln Thr Ala Leu Ser
    130                 135                 140
Ala Leu Gln Ser Phe Leu Gly Thr Ala Ile Ala Gly Met Asp Leu Asp
145                 150                 155                 160
Ser Leu Leu Arg Arg Arg Arg Asn Gly Glu Asp Val Ser Gly Ser Glu
                165                 170                 175
Leu Ala Lys Ala Gly Val Asp Leu Ala Ala Gln Leu Val Asp Asn Ile
            180                 185                 190
Ala Ser Ala Thr Gly Thr Val Asp Ala Phe Ala Glu Gln Leu Gly Lys
        195                 200                 205
Leu Gly Asn Ala Leu Ser Asn Thr Arg Leu Ser Gly Leu Ala Ser Lys
    210                 215                 220
Leu Asn Asn Leu Pro Asp Leu Ser Leu Ala Gly Pro Gly Phe Asp Ala
225                 230                 235                 240
Val Ser Gly Ile Leu Ser Val Val Ser Ala Ser Phe Ile Leu Ser Asn
                245                 250                 255
Lys Asp Ala Asp Ala Gly Thr Lys Ala Ala Ala Gly Ile Glu Ile Ser
            260                 265                 270
Thr Lys Ile Leu Gly Asn Ile Gly Lys Ala Val Ser Gln Tyr Ile Ile
        275                 280                 285
Ala Gln Arg Val Ala Ala Gly Leu Ser Thr Thr Ala Ala Thr Gly Gly
    290                 295                 300
Leu Ile Gly Ser Val Val Ala Leu Ala Ile Ser Pro Leu Ser Phe Leu
305                 310                 315                 320
Asn Val Ala Asp Lys Phe Glu Arg Ala Lys Gln Leu Glu Gln Tyr Ser
                325                 330                 335
Glu Arg Phe Lys Lys Phe Gly Tyr Glu Gly Asp Ser Leu Leu Ala Ser
            340                 345                 350
Phe Tyr Arg Glu Thr Gly Ala Ile Glu Ala Ala Leu Thr Thr Ile Asn
        355                 360                 365
Ser Val Leu Ser Ala Ala Ser Ala Gly Val Gly Ala Ala Ala Thr Gly
    370                 375                 380
Ser Leu Val Gly Ala Pro Val Ala Ala Leu Val Ser Ala Ile Thr Gly
385                 390                 395                 400
Ile Ile Ser Gly Ile Leu Asp Ala Ser Lys Gln Ala Ile Phe Glu Arg
                405                 410                 415
Val Ala Thr Lys Leu Ala Asn Lys Ile Asp Glu Trp Glu Lys Lys His
            420                 425                 430
Gly Lys Asn Tyr Phe Glu Asn Gly Tyr Asp Ala Arg His Ser Ala Phe
        435                 440                 445
Leu Glu Asp Thr Phe Glu Leu Leu Ser Gln Tyr Asn Lys Glu Tyr Ser
    450                 455                 460
Val Glu Arg Val Val Ala Ile Thr Gln Gln Arg Trp Asp Val Asn Ile
465                 470                 475                 480
Gly Glu Leu Ala Gly Ile Thr Arg Lys Gly Ala Asp Ala Lys Ser Gly
                485                 490                 495
Lys Ala Tyr Val Asp Phe Phe Glu Glu Gly Lys Leu Leu Glu Lys Asp
            500                 505                 510
Pro Asp Arg Phe Asp Lys Lys Val Phe Asp Pro Leu Glu Gly Lys Ile
        515                 520                 525
Asp Leu Ser Ser Ile Asn Lys Thr Thr Leu Leu Lys Phe Ile Thr Pro
    530                 535                 540
Val Phe Thr Ala Gly Glu Glu Ile Arg Glu Arg Lys Gln Thr Gly Ala

545                 550                 555                 560
Tyr Glu Tyr Met Thr Glu Leu Phe Val Lys Gly Lys Glu Lys Trp Val
                565                 570                 575
Val Thr Gly Val Gln Ser His Asn Ala Ile Tyr Asp Tyr Thr Asn Leu
            580                 585                 590
Ile Gln Leu Ala Ile Asp Lys Lys Gly Glu Lys Arg Gln Val Thr Ile
        595                 600                 605
Glu Ser His Leu Gly Glu Lys Asn Asp Arg Ile Tyr Leu Ser Ser Gly
    610                 615                 620
Ser Ser Ile Val Tyr Ala Gly Asn Gly His Asp Val Ala Tyr Tyr Asp
625                 630                 635                 640
Lys Thr Asp Thr Gly Tyr Leu Thr Phe Asp Gly Gln Ser Ala Gln Lys
                645                 650                 655
Ala Gly Glu Tyr Ile Val Thr Lys Glu Leu Lys Ala Asp Val Lys Val
            660                 665                 670
Leu Lys Glu Val Val Lys Thr Gln Asp Ile Ser Val Gly Ala Arg Ser
        675                 680                 685
Glu Lys Leu Glu Tyr Arg Asp Tyr Glu Leu Ser Pro Phe Glu Leu Gly
    690                 695                 700
Asn Gly Ile Arg Ala Lys Asp Glu Leu His Ser Val Glu Glu Ile Ile
705                 710                 715                 720
Gly Ser Asn Arg Lys Asp Lys Phe Phe Gly Ser Arg Phe Thr Asp Ile
                725                 730                 735
Phe His Gly Ala Lys Gly Asp Asp Glu Ile Tyr Gly Asn Asp Gly His
            740                 745                 750
Asp Ile Leu Tyr Gly Asp Asp Gly Asn Asp Val Ile His Gly Gly Asp
        755                 760                 765
Gly Asn Asp His Leu Val Gly Gly Asn Gly Asn Asp Arg Leu Ile Gly
    770                 775                 780
Gly Lys Gly Asn Asn Phe Leu Asn Gly Gly Asp Gly Asp Asp Glu Leu
785                 790                 795                 800
Gln Val Phe Glu Gly Gln Tyr Asn Val Leu Leu Gly Gly Ala Gly Asn
                805                 810                 815
Asp Ile Leu Tyr Gly Ser Asp Gly Thr Asn Leu Phe Asp Gly Gly Val
            820                 825                 830
Gly Asn Asp Lys Ile Tyr Gly Gly Leu Gly Lys Asp Ile Tyr Arg Tyr
        835                 840                 845
Ser Lys Glu Tyr Gly Arg His Ile Ile Ile Glu Lys Gly Gly Asp Asp
    850                 855                 860
Asp Thr Leu Leu Leu Ser Asp Leu Ser Phe Lys Asp Val Gly Phe Ile
865                 870                 875                 880
Arg Ile Gly Asp Asp Leu Leu Val Asn Lys Arg Ile Gly Gly Thr Leu
                885                 890                 895
Tyr Tyr His Glu Asp Tyr Asn Gly Asn Ala Leu Thr Ile Lys Asp Trp
            900                 905                 910
Phe Lys Glu Gly Lys Glu Gly Gln Asn Asn Lys Ile Glu Lys Ile Val
        915                 920                 925
Asp Lys Asp Gly Ala Tyr Val Leu Ser Gln Tyr Leu Thr Glu Leu Thr
    930                 935                 940
Ala Pro Gly Arg Gly Ile Asn Tyr Phe Asn Gly Leu Glu Glu Lys Leu
945                 950                 955                 960
Tyr Tyr Gly Glu Gly Tyr Asn Ala Leu Pro Gln Leu Arg Lys Asp Ile
                965                 970                 975
Glu Gln Ile Ile Ser Ser Thr Gly Ala Leu Thr Gly Glu His Gly Gln
            980                 985                 990
Val Leu Val Gly Ala Gly Gly Pro Leu Ala Tyr Ser Asn Ser Pro Asn
        995                 1000                1005
Ser Ile Pro Asn Ala Phe Ser Asn Tyr Leu Thr Gln Ser Ala
    1010                1015                1020

<210> 5
<211> 956
<212> PRT

<213> Actonbacillus pleuropneumoniae

<220>
<223> ApxIIA S148G K557A N687A

<400> 5
Met Ser Lys Ile Thr Leu Ser Ser Leu Lys Ser Ser Leu Gln Gln Gly
1               5                   10                  15
Leu Lys Asn Gly Lys Asn Lys Leu Asn Gln Ala Gly Thr Thr Leu Lys
            20                  25                  30
Asn Gly Leu Thr Gln Thr Gly His Ser Leu Gln Asn Gly Ala Lys Lys
        35                  40                  45
Leu Ile Leu Tyr Ile Pro Gln Gly Tyr Asp Ser Gly Gln Gly Asn Gly
    50                  55                  60
Val Gln Asp Leu Val Lys Ala Ala Asn Asp Leu Gly Ile Glu Val Trp
65                  70                  75                  80
Arg Glu Glu Arg Ser Asn Leu Asp Ile Ala Lys Thr Ser Phe Asp Thr
                85                  90                  95
Thr Gln Lys Ile Leu Gly Phe Thr Asp Arg Gly Ile Val Leu Phe Ala
            100                 105                 110
Pro Gln Leu Asp Asn Leu Leu Lys Lys Asn Pro Lys Ile Gly Asn Thr
        115                 120                 125
Leu Gly Ser Ala Ser Ser Ile Ser Gln Asn Ile Gly Lys Ala Asn Thr
    130                 135                 140
Val Leu Gly Gly Ile Gln Ser Ile Leu Gly Ser Val Leu Ser Gly Val
145                 150                 155                 160
Asn Leu Asn Glu Leu Leu Gln Asn Lys Asp Pro Asn Gln Leu Glu Leu
                165                 170                 175
Ala Lys Ala Gly Leu Glu Leu Thr Asn Glu Leu Val Gly Asn Ile Ala
            180                 185                 190
Ser Ser Val Gln Thr Val Asp Ala Phe Ala Glu Gln Ile Ser Lys Leu
        195                 200                 205
Gly Ser His Leu Gln Asn Val Lys Gly Leu Gly Gly Leu Ser Asn Lys
    210                 215                 220
Leu Gln Asn Leu Pro Asp Leu Gly Lys Ala Ser Leu Gly Leu Asp Ile
225                 230                 235                 240
Ile Ser Gly Leu Leu Ser Gly Ala Ser Ala Gly Leu Ile Leu Ala Asp
                245                 250                 255
Lys Glu Ala Ser Thr Glu Lys Lys Ala Ala Ala Gly Val Glu Phe Ala
            260                 265                 270
Asn Gln Ile Ile Gly Asn Val Thr Lys Ala Val Ser Ser Tyr Ile Leu
        275                 280                 285
Ala Gln Arg Val Ala Ser Gly Leu Ser Ser Thr Gly Pro Val Ala Ala
    290                 295                 300
Leu Ile Ala Ser Thr Val Ala Leu Ala Val Ser Pro Leu Ser Phe Leu
305                 310                 315                 320
Asn Val Ala Asp Lys Phe Lys Gln Ala Asp Leu Ile Lys Ser Tyr Ser
                325                 330                 335
Glu Arg Phe Gln Lys Leu Gly Tyr Asp Gly Asp Arg Leu Leu Ala Asp
            340                 345                 350
Phe His Arg Glu Thr Gly Thr Ile Asp Ala Ser Val Thr Thr Ile Asn
        355                 360                 365
Thr Ala Leu Ala Ala Ile Ser Gly Gly Val Gly Ala Ala Ser Ala Gly
    370                 375                 380
Ser Leu Val Gly Ala Pro Val Ala Leu Leu Val Ala Gly Val Thr Gly
385                 390                 395                 400
Leu Ile Thr Thr Ile Leu Glu Tyr Ser Lys Gln Ala Met Phe Glu His
                405                 410                 415
Val Ala Asn Lys Val His Asp Arg Ile Val Glu Trp Glu Lys Lys His
            420                 425                 430
Asn Lys Asn Tyr Phe Glu Gln Gly Tyr Asp Ser Arg His Leu Ala Asp
        435                 440                 445

Leu Gln Asp Asn Met Lys Phe Leu Ile Asn Leu Asn Lys Glu Leu Gln
    450                 455                 460
Ala Glu Arg Val Val Ala Ile Thr Gln Gln Arg Trp Asp Asn Gln Ile
465                 470                 475                 480
Gly Asp Leu Ala Ala Ile Ser Arg Arg Thr Asp Lys Ile Ser Ser Gly
                485                 490                 495
Lys Ala Tyr Val Asp Ala Phe Glu Glu Gly Gln His Gln Ser Tyr Asp
            500                 505                 510
Ser Ser Val Gln Leu Asp Asn Lys Asn Gly Ile Ile Asn Ile Ser Asn
        515                 520                 525
Thr Asn Arg Lys Thr Gln Ser Val Leu Phe Arg Thr Pro Leu Leu Thr
    530                 535                 540
Pro Gly Glu Glu Asn Arg Glu Arg Ile Gln Glu Gly Ala Asn Ser Tyr
545                 550                 555                 560
Ile Thr Lys Leu His Ile Gln Arg Val Asp Ser Trp Thr Val Thr Asp
                565                 570                 575
Gly Asp Ala Ser Ser Ser Val Asp Phe Thr Asn Val Val Gln Arg Ile
            580                 585                 590
Ala Val Lys Phe Asp Asp Ala Gly Asn Ile Ile Glu Ser Lys Asp Thr
        595                 600                 605
Lys Ile Ile Ala Asn Leu Gly Ala Gly Asn Asp Asn Val Phe Val Gly
    610                 615                 620
Ser Ser Thr Thr Val Ile Asp Gly Gly Asp Gly His Asp Arg Val His
625                 630                 635                 640
Tyr Ser Arg Gly Glu Tyr Gly Ala Leu Val Ile Asp Ala Thr Ala Glu
                645                 650                 655
Thr Glu Lys Gly Ser Tyr Ser Val Lys Arg Tyr Val Gly Asp Ser Lys
            660                 665                 670
Ala Leu His Glu Thr Ile Ala Thr His Gln Thr Asn Val Gly Ala Arg
        675                 680                 685
Glu Glu Lys Ile Glu Tyr Arg Arg Glu Asp Asp Arg Phe His Thr Gly
    690                 695                 700
Tyr Thr Val Thr Asp Ser Leu Lys Ser Val Glu Glu Ile Ile Gly Ser
705                 710                 715                 720
Gln Phe Asn Asp Ile Phe Lys Gly Ser Gln Phe Asp Asp Val Phe His
                725                 730                 735
Gly Gly Asn Gly Val Asp Thr Ile Asp Gly Asn Asp Gly Asp Asp His
            740                 745                 750
Leu Phe Gly Gly Ala Gly Asp Asp Val Ile Asp Gly Gly Asn Gly Asn
        755                 760                 765
Asn Phe Leu Val Gly Gly Thr Gly Asn Asp Ile Ile Ser Gly Gly Lys
    770                 775                 780
Asp Asn Asp Ile Tyr Val His Lys Thr Gly Asp Gly Asn Asp Ser Ile
785                 790                 795                 800
Thr Asp Ser Gly Gly Gln Asp Lys Leu Ala Phe Ser Asp Val Asn Leu
                805                 810                 815
Lys Asp Leu Thr Phe Lys Lys Val Asp Ser Ser Leu Glu Ile Ile Asn
            820                 825                 830
Gln Lys Gly Glu Lys Val Arg Ile Gly Asn Trp Phe Leu Glu Asp Asp
        835                 840                 845
Leu Ala Ser Thr Val Ala Asn Tyr Lys Ala Thr Asn Asp Arg Lys Ile
    850                 855                 860
Glu Glu Ile Ile Gly Lys Gly Gly Glu Arg Ile Thr Ser Glu Gln Val
865                 870                 875                 880
Asp Lys Leu Ile Lys Glu Gly Asn Asn Gln Ile Ser Ala Glu Ala Leu
                885                 890                 895
Ser Lys Val Val Asn Asp Tyr Asn Thr Ser Lys Asp Arg Gln Asn Val
            900                 905                 910
Ser Asn Ser Leu Ala Lys Leu Ile Ser Ser Val Gly Ser Phe Thr Ser
        915                 920                 925
Ser Ser Asp Phe Arg Asn Asn Leu Gly Thr Tyr Val Pro Ser Ser Ile
    930                 935                 940
Asp Val Ser Asn Asn Ile Gln Leu Ala Arg Ala Ala

945                 950                 955

<210> 6
<211> 1052
<212> PRT
<213> Actonbacillus pleuropneumoniae

<220>
<223> ApxIIIA K571A K702A

<400> 6
Met Ser Thr Trp Ser Ser Met Leu Ala Asp Leu Lys Lys Arg Ala Glu
1               5                   10                  15
Glu Ala Lys Arg Gln Ala Lys Lys Gly Tyr Asp Val Thr Lys Asn Gly
            20                  25                  30
Leu Gln Tyr Gly Val Ser Gln Ala Lys Leu Gln Ala Leu Ala Ala Gly
        35                  40                  45
Lys Ala Val Gln Lys Tyr Gly Asn Lys Leu Val Leu Val Ile Pro Lys
    50                  55                  60
Glu Tyr Asp Gly Ser Val Gly Asn Gly Phe Phe Asp Leu Val Lys Ala
65                  70                  75                  80
Ala Glu Glu Leu Gly Ile Gln Val Lys Tyr Val Asn Arg Asn Glu Leu
                85                  90                  95
Glu Val Ala His Lys Ser Leu Gly Thr Ala Asp Gln Phe Leu Gly Leu
            100                 105                 110
Thr Glu Arg Gly Leu Thr Leu Phe Ala Pro Gln Leu Asp Gln Phe Leu
        115                 120                 125
Gln Lys His Ser Lys Ile Ser Asn Val Val Gly Ser Ser Thr Gly Asp
    130                 135                 140
Ala Val Ser Lys Leu Ala Lys Ser Gln Thr Ile Ile Ser Gly Ile Gln
145                 150                 155                 160
Ser Val Leu Gly Thr Val Leu Ala Gly Ile Asn Leu Asn Glu Ala Ile
                165                 170                 175
Ile Ser Gly Gly Ser Glu Leu Glu Leu Ala Glu Ala Gly Val Ser Leu
            180                 185                 190
Ala Ser Glu Leu Val Ser Asn Ile Ala Lys Gly Thr Thr Thr Ile Asp
        195                 200                 205
Ala Phe Thr Thr Gln Ile Gln Asn Phe Gly Lys Leu Val Glu Asn Ala
    210                 215                 220
Lys Gly Leu Gly Gly Val Gly Arg Gln Leu Gln Asn Ile Ser Gly Ser
225                 230                 235                 240
Ala Leu Ser Lys Thr Gly Leu Gly Leu Asp Ile Ile Ser Ser Leu Leu
                245                 250                 255
Ser Gly Val Thr Ala Ser Phe Ala Leu Ala Asn Lys Asn Ala Ser Thr
            260                 265                 270
Ser Thr Lys Val Ala Ala Gly Phe Glu Leu Ser Asn Gln Val Ile Gly
        275                 280                 285
Gly Ile Thr Lys Ala Val Ser Ser Tyr Ile Leu Ala Gln Arg Leu Ala
    290                 295                 300
Ala Gly Leu Ser Thr Thr Gly Pro Ala Ala Ala Leu Ile Ala Ser Ser
305                 310                 315                 320
Ile Ser Leu Ala Ile Ser Pro Leu Ala Phe Leu Arg Val Ala Asp Asn
                325                 330                 335
Phe Asn Arg Ser Lys Glu Ile Gly Glu Phe Ala Glu Arg Phe Lys Lys
            340                 345                 350
Leu Gly Tyr Asp Gly Asp Lys Leu Leu Ser Glu Phe Tyr His Glu Ala
        355                 360                 365
Gly Thr Ile Asp Ala Ser Ile Thr Thr Ile Ser Thr Ala Leu Ser Ala
    370                 375                 380
Ile Ala Ala Gly Thr Ala Ala Ala Ser Ala Gly Ala Leu Val Gly Ala
385                 390                 395                 400
Pro Ile Thr Leu Leu Val Thr Gly Ile Thr Gly Leu Ile Ser Gly Ile

                405                 410                 415
Leu Glu Phe Ser Lys Gln Pro Met Leu Asp His Val Ala Ser Lys Ile
            420                 425                 430
Gly Asn Lys Ile Asp Glu Trp Glu Lys Lys Tyr Gly Lys Asn Tyr Phe
        435                 440                 445
Glu Asn Gly Tyr Asp Ala Arg His Lys Ala Phe Leu Glu Asp Ser Phe
    450                 455                 460
Ser Leu Leu Ser Ser Phe Asn Lys Gln Tyr Glu Thr Glu Arg Ala Val
465                 470                 475                 480
Leu Ile Thr Gln Gln Arg Trp Asp Glu Tyr Ile Gly Glu Leu Ala Gly
                485                 490                 495
Ile Thr Gly Lys Gly Asp Lys Leu Ser Ser Gly Lys Ala Tyr Val Asp
            500                 505                 510
Tyr Phe Gln Glu Gly Lys Leu Leu Glu Lys Lys Pro Asp Asp Phe Ser
        515                 520                 525
Lys Val Val Phe Asp Pro Thr Lys Gly Glu Ile Asp Ile Ser Asn Ser
    530                 535                 540
Gln Thr Ser Thr Leu Leu Lys Phe Val Thr Pro Leu Leu Thr Pro Gly
545                 550                 555                 560
Thr Glu Ser Arg Glu Arg Thr Gln Thr Gly Ala Tyr Glu Tyr Ile Thr
                565                 570                 575
Lys Leu Val Val Lys Gly Lys Asp Lys Trp Val Val Asn Gly Val Lys
            580                 585                 590
Asp Lys Gly Ala Val Tyr Asp Tyr Thr Asn Leu Ile Gln His Ala His
        595                 600                 605
Ile Ser Ser Ser Val Ala Arg Gly Glu Glu Tyr Arg Glu Val Arg Leu
    610                 615                 620
Val Ser His Leu Gly Asn Gly Asn Asp Lys Val Phe Leu Ala Ala Gly
625                 630                 635                 640
Ser Ala Glu Ile His Ala Gly Glu Gly His Asp Val Val Tyr Tyr Asp
                645                 650                 655
Lys Thr Asp Thr Gly Leu Leu Val Ile Asp Gly Thr Lys Ala Thr Glu
            660                 665                 670
Gln Gly Arg Tyr Ser Val Thr Arg Glu Leu Ser Gly Ala Thr Lys Ile
        675                 680                 685
Leu Arg Glu Val Ile Lys Asn Gln Lys Ser Ala Val Gly Ala Arg Glu
    690                 695                 700
Glu Thr Leu Glu Tyr Arg Asp Tyr Glu Leu Thr Gln Ser Gly Asn Ser
705                 710                 715                 720
Asn Leu Lys Ala His Asp Glu Leu His Ser Val Glu Glu Ile Ile Gly
                725                 730                 735
Ser Asn Gln Arg Asp Glu Phe Lys Gly Ser Lys Phe Arg Asp Ile Phe
            740                 745                 750
His Gly Ala Asp Gly Asp Asp Leu Leu Asn Gly Asn Asp Gly Asp Asp
        755                 760                 765
Ile Leu Tyr Gly Asp Lys Gly Asn Asp Glu Leu Arg Gly Asp Asn Gly
    770                 775                 780
Asn Asp Gln Leu Tyr Gly Gly Glu Gly Asn Asp Lys Leu Leu Gly Gly
785                 790                 795                 800
Asn Gly Asn Asn Tyr Leu Ser Gly Gly Asp Gly Asn Asp Glu Leu Gln
                805                 810                 815
Val Leu Gly Asn Gly Phe Asn Val Leu Arg Gly Gly Lys Gly Asp Asp
            820                 825                 830
Lys Leu Tyr Gly Ser Ser Gly Ser Asp Leu Leu Asp Gly Gly Glu Gly
        835                 840                 845
Asn Asp Tyr Leu Glu Gly Gly Asp Gly Ser Asp Phe Tyr Val Tyr Arg
    850                 855                 860
Ser Thr Ser Gly Asn His Thr Ile Tyr Asp Gln Gly Lys Ser Ser Asp
865                 870                 875                 880
Leu Asp Lys Leu Tyr Leu Ser Asp Phe Ser Phe Asp Arg Leu Leu Val
                885                 890                 895
Glu Lys Val Asp Asp Asn Leu Val Leu Arg Ser Asn Glu Ser Ser His
            900                 905                 910

Asn Asn Gly Val Leu Thr Ile Lys Asp Trp Phe Lys Glu Gly Asn Lys
        915                 920                 925
Tyr Asn His Lys Ile Glu Gln Ile Val Asp Lys Asn Gly Arg Lys Leu
    930                 935                 940
Thr Ala Glu Asn Leu Gly Thr Tyr Phe Lys Asn Ala Pro Lys Ala Asp
945                 950                 955                 960
Asn Leu Leu Asn Tyr Ala Thr Lys Glu Asp Gln Asn Glu Ser Asn Leu
                965                 970                 975
Ser Ser Leu Lys Thr Glu Leu Ser Lys Ile Ile Thr Asn Ala Gly Asn
            980                 985                 990
Phe Gly Val Ala Lys Gln Gly Asn Thr Gly Ile Asn Thr Ala Ala Leu
        995                 1000                1005
Asn Asn Glu Val Asn Lys Ile Ile Ser Ser Ala Asn Thr Phe Ala Thr
    1010                1015                1020
Ser Gln Leu Gly Gly Ser Gly Met Gly Thr Leu Pro Ser Thr Asn Val
1025                1030                1035                1040
Asn Ser Met Met Leu Gly Asn Leu Ala Arg Ala Ala
                1045                1050

<210> 7
<211> 3078
<212> DNA
<213> Actonbacillus pleuropneumoniae

<220>
<223> ApxIA wild type

<400> 7
ggatccgcaa attctcaact ggaccgcgta aagggcctca ttgacagcct caaccaacac      60

accaaatcgg ctgcgaaatc cggtgcgggt gcgctgaaaa atggtctggg tcaggtgaaa     120

caggcgggtc agaaactgat cttatacatc ccgaaagatt atcaggcttc aaccggctcc     180

agtcttaatg atttggtaaa agccgccgaa gcactgggta ttgaagtaca tcgcagcgaa     240

aaaaatggga ctgcgttagc gaaagagctg tttggcacaa cggaaaagct gctgggcttt     300

agtgaacgtg gcattgcgct gtttgcacca cagttcgata aattgttaaa taagaatcag     360

aaactgagca agtccttagg cggctcatca gaagccctgg gtcagcgtct gaacaaaact     420

cagacagccc tgagtgcatt acagtcattt ctcgggacgg caattgcagg catggatctg     480

gatagcctgc ttcgccgtcg gcgtaacggt gaggatgtct cgggcagcga gctggcaaaa     540

gccggcgttg acctggcagc ccagttggtt gacaacatcg catctgccac cggcactgtt     600

gacgcttttg ccgaacaact gggcaaatta gggaacgcgc tgtctaacac gcgcttatct     660

gggctcgcat ccaaactcaa caacttgccg gacttgtcgc tggcaggacc tggctttgac     720

gcggtttcag gcattctgtc cgtggttagt gcttcgttca ttcttagcaa caaagacgcc     780

gatgcgggta ccaaggcggc cgctgggatt gaaatcagca cgaaaatctt gggtaatatt     840

ggcaaggcag tgtcgcagta tatcatcgct caacgtgtgg ccgcaggcct cagcaccacc     900

gcggccacag gtggcctgat tggtagtgtc gtcgctctgg ccatttctcc cctgtcgttc     960

ctgaacgttg cggacaaatt tgaacgcgct aaacagctgg aacagtattc tgaacgcttt    1020

aaaaaatttg gatatgaagg cgatagcctg ctggcatcct tttaccgcga aaccggtgcg 1080

attgaagccg cattgaccac cattaactcg gtactcagtg ctgccagtgc aggggtggga 1140

gcggcagcca caggttctct ggtcggagcc ccggttgctg ccttagtgtc tgccattacc 1200

gggattatct cgggcatcct tgatgcgtct aaacaggcga ttttcgaacg cgtggcgacg 1260

aaactggcga acaaaattga tgaatgggaa aagaaacacg gtaagaacta ctttgagaac 1320

ggctatgatg cccgtcatag tgcgtttctc gaagatacct ttgaactttt gtcgcaatac 1380

aacaaagagt atagcgtgga gcgcgtagtt gctattacac agcagcgttg ggatgtgaat 1440

attggggaat tggcggggat cacccgtaaa ggcgcggatg caaaatccgg taaagcttac 1500

gtcgatttct tcgaggaagg gaaactgctg gaaaaggacc cggatcgctt tgataagaaa 1560

gtgttcgacc cgcttgaggg taaaattgac ttgtcatcca ttaacaaaac gacgttgctg 1620

aaattcatta cgcccgtgtt tacggcggga gaagaaattc gcgaacgcaa acagactggg 1680

aaatacgagt acatgactga actgtttgtt aaagggaagg aaaagtgggt agtcaccggt 1740

gttcagtcgc acaatgcgat ttacgattat actaacctga ttcagctggc aatcgacaag 1800

aaaggtgaaa aacgccaagt gaccatcgag agtcaccttg gcgaaaagaa cgatcgtatc 1860

tatctgagca gtggcagctc aatcgtctat gccggcaacg gtcatgacgt tgcctattac 1920

gataaaacgg ataccggata tctgacgttt gacggtcaaa gcgcgcagaa agcgggtgaa 1980

tatatcgtca ctaaggaatt gaaagccgac gttaaagtcc tgaaagaggt ggtcaaaacc 2040

caagatatct cagtaggcaa acgcagcgag aaactggagt accgcgatta tgagctgagc 2100

ccgtttgaac ttggtaacgg gattcgtgcg aaagatgaac tgcatagcgt ggaagagatt 2160

attggatcga atcggaaaga caaattcttt ggtagccgct tcaccgatat ctttcatggc 2220

gccaaaggcg acgacgagat ttatgggaat gatgggcatg atatcctgta tggcgacgat 2280

ggcaacgatg tgattcatgg aggcgacggt aatgatcact tagtgggcgg caatggaaat 2340

gaccgtctga tcggcgggaa aggcaataat ttcctgaatg gcggtgatgg cgatgatgaa 2400

cttcaggtgt tcgaggggca atataatgtg ctgttaggcg gcgccggcaa cgatatttta 2460

tacggtagtg atggtaccaa cctcttcgat ggtggcgtgg gtaatgataa aatctatggt 2520

ggcctgggca aagatatcta tcgctattct aaagaatacg gtcggcatat tatcattgaa 2580

aaaggtggtg acgacgacac cctgctgttg agcgatcttt ccttcaagga cgtcggtttc 2640

attcgtattg gagatgatct gctggtgaac aaacggatcg gcggcaccct gtattaccac 2700

gaggattaca acggtaatgc gcttacaatc aaagattggt ttaaagaggg taaggaaggt 2760

cagaataaca aaattgaaaa gatcgttgac aaagatggag cttatgtcct gtcccagtat 2820

ttgaccgaac tcacggcgcc tggtcgtggc attaattact tcaacggatt agaagagaag 2880

ttatactacg gtgaaggcta caacgcgctc ccgcaactgc gcaaagatat tgaacaaatc 2940

atctccagca ccggagcttt aacgggcgaa catggccaag ttctggtagg agcgggtggc 3000

ccactggcgt attcaaattc tccgaacagc attccaaacg cgttttcgaa ttatctgact 3060

cagagcgctt aagagctc 3078

<210> 8
<211> 2880
<212> DNA
<213> Actonbacillus pleuropneumoniae

<220>
<223> ApxIIA wild type

<400> 8
ggatcctcga aaattacctt gtccagcctg aaatccagct tacagcaagg tctgaagaat 60

gggaagaaca aactgaacca agccggtacc acgctgaaga atggattgac gcaaacaggt 120

catagtctgc agaatggtgc gaagaaatta atcctctaca ttccgcaggg ctatgactca 180

ggccaaggca acggggtcca ggacctcgta aaagccgcga atgatctggg cattgaggtg 240

tggcgcgaag aacgtagtaa cctggatatt gcgaaaactt cgttcgatac cacccagaaa 300

attctgggat tcaccgaccg gggcatcgtg ttgttcgcac cccagctgga taatctcctg 360

aagaagaacc cgaaaatcgg caacacgctg ggcagcgcat caagcatctc gcagaacatc 420

ggcaaagcca ataccgtctt aggtggaatc cagagcattc tgggtagcgt gctttctggc 480

gtgaacttaa acgaattact tcagaacaaa gacccgaatc agctggaact ggcgaaagct 540

ggactggaac ttacgaacga actggtaggt aacattgcca gctctgtaca aaccgttgat 600

gcgtttgcgg agcagatcag taaactgggt tctcatttgc aaaatgttaa agggctgggt 660

ggtctgagta acaaattgca gaatctgccg gacctgggca aggcttcttt ggggctggat 720

attatctcgg ggctgttgtc tggcgcaagc gctggcctga ttttagcgga taaggaggct 780

tcgaccgaaa agaaggccgc tgccggcgta gaatttgcga accagattat tgggaacgtg 840

accaaagcgg tgagtagcta tattttggcg caacgtgttg cctccggttt gagtagtact 900

ggtccagtcg cagcactgat tgccagtact gtggcactgg cggttagtcc actgtccttt 960

ctgaatgtcg cggataaatt taaacaagcc gatctcatca aatcttatag tgaacgtttc 1020

cagaaattag gttatgatgg cgatcgctta ctggcggact ttcaccggga aacaggcacc 1080

attgatgcaa gcgtgaccac gattaatacc gcgcttgcag caatcagcgg tggtgttggg 1140

gcagcgagtg ctggaagcct ggttggcgcg cctgttgctc tgcttgtagc gggtgtaacg 1200

ggcttaatca caacgatcct cgaatattcc aaacaagcga tgtttgagca tgtcgctaac 1260

aaagtccacg accgcatcgt ggaatgggaa aaaaaacaca ataaaaacta ctttgagcag 1320

ggctacgaca gccgtcattt ggcagatctg caggacaata tgaaattcct catcaactta 1380

aacaaagaac ttcaggctga acgtgtcgtg gcgattacgc aacaacgttg ggataaccag 1440

attggcgacc tggcggccat ttcccgtcgc actgacaaaa tttcctcggg taaagcatat 1500

gtggacgcgt ttgaggaagg tcagcaccag agctatgata gttcagtgca gttagacaat 1560

aaaaatggta ttattaacat ttcaaacaca aaccgcaaga cccaatcagt actgtttcgc 1620

acgccgttac tgaccccggg tgaagaaaac cgcgagcgca ttcaggaggg taagaactcc 1680

tacattacca aacttcacat tcagcgcgta gattcatgga cggtgacgga tggcgatgcg 1740

tcatcgagcg tagatttcac caatgtggtt cagcgtattg cggtgaaatt cgatgatgca 1800

ggaaacatta tcgagtcaaa agatactaaa attatcgcca atctcggtgc cggtaatgac 1860

aatgtctttg taggcagcag cacgacggtg attgacggcg gtgacggcca tgatcgcgtt 1920

cattattcgc gtggcgaata cggcgctctg gtcattgacg caactgccga gacagagaaa 1980

ggtagctact ccgttaaacg ttatgttggt gactcaaaag cgctgcacga aacgattgcc 2040

actcatcaga ccaatgtggg gaatcgcgaa gagaaaatcg agtatcgccg cgaagatgat 2100

cggttccata ccgggtatac tgtgaccgat tcgctgaaaa gcgtcgaaga aattatcgga 2160

tctcaattca atgacatctt caaaggttcc cagtttgatg acgtgtttca tggcggtaat 2220

ggcgttgata ccattgacgg caacgatggc gacgaccatc tgtttggcgg ggctggtgat 2280

gacgtgatcg atggcggcaa cggcaacaac tttctggtgg gtggtaccgg gaacgatatc 2340

atttcaggcg gcaaagataa cgacatctac gttcacaaga caggggatgg gaatgatagc 2400

attactgatt cgggtggcca agataagctg gccttttctg acgttaatct gaaagatctg 2460

actttcaaaa aggtggattc tagcctggaa atcattaatc agaaaggaga gaaggtccgc 2520

attggaaatt ggtttctcga agatgatctt gcctcgaccg tggcaaacta caaagcgacc 2580

aatgaccgca aaatcgaaga aatcattggg aaaggcggtg aacgcatcac ctctgaacag 2640

gtggataaac tgatcaaaga aggcaataac cagatttccg ctgaagcgtt gtctaaagtc 2700

gtcaacgatt acaacacgtc caaagatcgc caaaacgtta gcaattcact tgccaaactg 2760

atctcgagcg ttggatcttt tacatcctcg agcgatttcc gtaacaacct cggcacctat 2820

gttccttctt cgattgatgt cagtaataat atccaactgg cccgtgccgc ctaagagctc 2880

<210> 9
<211> 3168
<212> DNA
<213> Actonbacillus pleuropneumoniae

<220>
<223> ApxIIIA wild type

<400> 9
ggatcctcta cctggtcctc catgctggct gatctgaaga agcgtgcgga agaagccaaa 60

cgtcaggcga aaaaaggtta tgacgtcacg aagaacggcc tgcagtacgg tgttagtcaa 120

gccaaactgc aggctctggc agccggtaaa gctgttcaga aatacggtaa caaacttgtg 180

ttagtaattc cgaaagaata tgatgggtcg gtgggtaatg gcttcttcga cctggtcaaa 240

gccgcggaag aactgggcat tcaggtgaaa tatgtcaacc gcaacgaact cgaggttgcg 300

cataaatctc tgggaacggc ggatcagttt ctgggtctga ctgagcgtgg cctgaccctg 360

tttgcaccgc agcttgacca gttcttacag aaacacagca aaatctcaaa tgtggttggt 420

tcttcaactg gtgacgcggt gagcaaactg gcaaagtctc agacaatcat ttctggaatc 480

cagtcggtgt tgggcactgt actcgctggg atcaacttaa atgaagccat catttcaggc 540

ggctctgaat tggagctggc ggaagctggc gtaagcctgg cgtcggaact ggtgtcgaat 600

atcgccaaag gcaccacgac aattgatgcg tttaccaccc aaattcagaa ttttggaaaa 660

ctggtcgaaa acgcgaaggg cctgggtggc gtgggtcgtc agctgcaaaa catttccggt 720

agcgccttga gcaaaactgg tttgggctta gacatcatca gcagcttgct gagtggagtc 780

acagcgagct ttgcgctggc aaacaagaat gcctctacct ccaccaaagt tgctgccggt 840

tttgaactgt cgaatcaagt tattggaggc attaccaaag cggtcagctc atatatcctg 900

gcccaacgtc tggcggcggg tttgagcacg actggacccg ctgcagcgct gattgcatct 960

agtattagtc tggccatcag cccgctggcg tttctgcgcg tagctgataa tttcaaccgc 1020

agcaaagaaa tcggtgagtt cgcggaacgc tttaagaaat taggctatga tggggataag 1080

ctgctgtctg aattttacca tgaggctggc accattgacg catctatcac gacaatttcg 1140

acagccctgt cagcgattgc cgcagggact gcagccgctt cagccggcgc cctggttggt 1200

gcaccgatta cgcttcttgt cacgggtatt accggcttga ttagcggcat cctggaattt 1260

tccaaacagc cgatgctgga ccatgtagca agtaaaattg gcaacaaaat cgacgagtgg 1320

gaaaagaaat atggtaagaa ctacttcgag aatggttatg atgctcgcca taaagccttc 1380

ttggaagatt cgttcagcct gctcagttcc ttcaataagc aatatgaaac cgaacgcgcc 1440

gtgctgatca cccaacagcg ctgggatgag tacattgggg aactggcggg tattacgggt 1500

aagggtgaca aattatcaag cgggaaggca tacgttgatt attttcagga gggtaaactt 1560

ctggagaaga aacccgacga tttcagcaaa gtagtgtttg atccgacaaa aggcgagatc 1620

gatatcagca attcccagac ctctaccctg ctgaaatttg tgacccctct cctgacccca 1680

ggaactgaaa gccgtgaacg cactcaaacg ggaaagtacg aatacattac taaactcgtg 1740

gtaaaaggga aagataaatg ggtcgtgaac ggcgtgaaag ataaaggcgc ggtatacgat 1800

tacaccaact taattcagca tgctcacatt tcctcttccg tggcgcgtgg cgaagagtat 1860

cgggaagtcc gcctggttag ccatttgggt aatggcaatg ataaagtctt tctggctgcg 1920

ggctcggcag aaattcacgc cggcgaaggc catgatgtcg tatattacga taaaacggac 1980

accggactcc tggtgatcga tggcacaaaa gcaaccgagc aagggcgcta tagtgttacg 2040

cgtgagctga gtggcgcgac caaaattttg cgtgaagtga tcaaaaatca gaaatcggca 2100

gttggcaagc gcgaagaaac gttagagtat cgcgattacg aactcacgca aagcggtaac 2160

agtaatctga aagcacacga cgaactgcat tctgttgaag aaattattgg cagcaatcag 2220

cgcgatgaat tcaaaggctc gaagtttcgt gatatctttc atggtgcaga cggggatgac 2280

ctcctgaacg gcaatgatgg ggatgacatt ttatatggag acaaagggaa cgatgaactg 2340

cgcggcgata acgggaacga ccaactgtac ggtggtgagg ggaatgataa actgttgggc 2400

gggaacggca ataactattt atcgggcgga gatggtaacg atgaacttca ggttctgggg 2460

aacggtttca acgtccttcg tggaggcaaa ggtgacgata aactttatgg tagcagcggc 2520

tccgacctcc ttgatggtgg agaaggcaac gattacttag aaggtggtga tgggagtgat 2580

ttctatgtgt atcggagcac cagtggcaat cacaccattt atgatcaggg taaatcctcc 2640

gatctggaca aattatacct gtcagatttc tcgtttgacc gcctcttggt ggagaaagtg 2700

gatgacaacc tcgttttgcg ttccaatgag agtagccaca ataatggcgt tttaaccatc 2760

aaagactggt ttaaagaagg gaacaagtac aaccacaaaa tcgagcagat tgtcgataag 2820

aatggccgca aactgacggc cgaaaatctg ggtacctatt ttaaaaacgc cccaaaagcg 2880

gacaaccttc tgaactatgc gaccaaagag gaccagaacg aatcaaattt atcttcattg 2940

aaaacggaac tgagtaaaat tatcactaat gcgggtaatt ttggcgttgc caaacaaggt 3000

aacaccggca ttaataccgc agcgcttaac aacgaagtga acaaaatcat ttcgtcagcg 3060

aacaccttcg ctacgagtca actcggtggc tcggggatgg gcacactgcc ttccacgaac 3120

gtgaatagca tgatgctggg caatcttgcc cgggcggcgt aagagctc 3168

<210> 10
<211> 3078
<212> DNA
<213> Actonbacillus pleuropneumoniae

<220>
<223> ApxIA K560A K686A

<400> 10
ggatccgcta attctcagtt agatcgtgtg aaaggattaa tcgatagcct gaaccagcac 60

accaaatccg cagcaaaatc aggagctggc gctctgaaga atggactggg ccaggtgaaa 120

caggcagggc agaaactgat tctctatatt ccgaaagact atcaagcttc caccggttcc 180

agcttgaatg atttggtgaa agcagcggaa gctctgggaa ttgaagtcca tcgtagcgag 240

aagaacggaa ccgcattggc gaaagagtta tttgggacca cggaaaaact gttaggcttt 300

agtgaacggg gtattgcact gtttgcacct cagtttgaca aactgcttaa caaaaaccag 360

aagttaagca aatcactggg tggcagctcc gaagccttgg gccagcgcct gaacaaaacc 420

cagacggcat tgagtgcact gcaaagtttt ctgggcacgg ctatcgccgg aatggatctg 480

gattctctcc tgcgtcggcg tcgcaacggt gaagatgtaa gtggatcgga gctggcgaaa 540

gctggtgtcg atctcgcggc acaattggtt gacaatattg cgtcggccac aggaacggtg 600

gatgcgtttg cggaacaact cggtaaactg ggcaacgcgt tatctaacac acgcttatcc 660

ggcttagcct ctaagctgaa taaccttccg gatctgtcct tggccggccc cggctttgat 720

gcggttagtg gcattcttag cgttgtgtcg gcgagtttca ttctgtccaa caaagatgca 780

gatgcgggca ccaaagccgc cgcgggcatt gaaatttcga cgaaaattct tggtaacatc 840

ggcaaggctg tgtctcagta tatcattgct caacgtgtgg ccgccgggtt aagcactaca 900

gcagcgacag gtggcctgat tggctctgtg gtagcactcg caattagtcc gctgtcattt 960

ctgaatgtcg cggataaatt tgaacgtgcc aaacaactgg aacagtatag cgaacgcttc 1020

aagaaatttg gttatgaagg ggatagtttg cttgcgagct tctaccgcga aaccggtgcc 1080

attgaagcgg ccctgaccac gatcaactcg gtgttgtcag ccgccagcgc cggggtcggt 1140

gcggcagcga cgggttcctt ggtaggtgca ccagttgcgg cgcttgtatc agccattact 1200

ggcatcatct ccggcattct ggatgccagc aagcaagcta tcttcgaacg tgtggcgacg 1260

aaactggcga acaaaattga tgaatgggag aaaaagcacg gcaaaaatta cttcgaaaac 1320

ggttatgatg cgcgccattc ggcgtttctg gaggatacat ttgaacttct gagccagtac 1380

aacaaagaat actctgtgga gcgtgtcgta gcgatcaccc agcaacggtg ggatgttaac 1440

attggtgaac tggccggcat tacccgcaaa ggcgctgacg cgaaatcagg caaagcatac 1500

gtcgacttct ttgaagaggg gaaactgttg gaaaaggacc cggaccgttt tgataagaaa 1560

gtctttgatc cgctggaagg gaaaattgat ctgtcctcta tcaacaaaac tacgttgctg 1620

aaattcatca cgcccgtttt cacagcgggt gaggaaattc gcgagcgtaa gcagactggg 1680

gcgtatgaat acatgaccga actgttcgtc aaaggtaaag agaaatgggt ggtgaccggc 1740

gttcagtcgc ataacgccat ctacgattat accaacttaa tccagctggc gattgacaaa 1800

aaaggtgaaa agcgtcaggt gaccattgag agccatctgg gcgagaagaa tgaccgcatc 1860

tatttaagca gtggtagttc gattgtctat gctggcaatg ggcatgatgt cgcctattat 1920

gacaaaaccg atacgggcta tttgaccttt gacggtcaga gcgcccagaa agcaggcgaa 1980

tatatcgtaa ccaaagaact gaaagctgat gtgaaagtcc tgaaagaggt tgtgaaaacg 2040

caagatatta gcgttggcgc ccgctcggag aaattagaat atcgcgatta tgagctgagc 2100

ccattcgaac tcggtaacgg cattcgcgcg aaagatgaac tccactctgt ggaagaaatt 2160

attggttcta atcgtaaaga taaattcttc gggtcacgct ttaccgatat ctttcatggc 2220

gcgaaagggg acgatgaaat ctatggcaac gacggccacg acattctgta tggtgatgat 2280

gggaacgacg ttatccatgg aggggatggc aatgatcatc tggttggcgg caatggaaat 2340

gaccgtctta tcggcggcaa aggtaataac tttcttaacg gtggtgatgg ggacgacgaa 2400

cttcaggtct ttgaaggtca gtataacgta ctgctgggcg gtgctgggaa cgacatcctt 2460

tatggtagtg atggtactaa tctgttcgac ggcggcgtgg gtaacgacaa gatctacgga 2520

gggttgggta aggacattta ccgctactcg aaagaatacg gccggcacat cattattgag 2580

aaaggcggag atgacgatac cctcctgctg tcggatttaa gctttaaaga cgtgggcttc 2640

attcgcatcg gggatgatct gttagtgaac aaacgcattg gaggcacgct gtactaccac 2700

gaagattaca atggcaatgc tctgaccatt aaggactggt tcaaagaagg caaggaaggt 2760

caaaacaata agatcgagaa aatcgtagat aaagacggcg cctatgttct cagccagtat 2820

ctcactgaac tgactgcacc tggccgcggt attaattact tcaatggtct ggaagagaaa 2880

ctgtactatg gcgagggtta taacgccctg ccacaactgc gcaaagacat tgaacagatt 2940

atcagctcta ccggggcgct gactggtgag catggtcaag ttctcgttgg tgcgggtggt 3000

ccgctggcgt actcaaattc gccgaatagc atcccgaatg ccttttccaa ttacctgacc 3060

cagtcagcat aagagctc 3078

<210> 11
<211> 2880
<212> DNA
<213> Actonbacillus pleuropneumoniae

<220>
<223> ApxIIA S148A K557A K686A

<400> 11
ggatccagca aaattaccct ctcatccctc aaatcaagcc tgcagcaggg gctgaagaac 60

ggcaagaaca agctgaatca ggcgggtacg actctgaaga atggcttaac gcaaacaggg 120

cactcattac agaacggtgc gaaaaaactg atcctgtaca tcccgcaagg ttacgactcg 180

gggcagggca atggggtcca agatcttgtt aaagcggcca atgacctggg aatcgaagtg 240

tggcgtgaag aacggagcaa cctggacatt gcgaaaacca gctttgatac gacccagaaa 300

atcctgggtt tcaccgaccg cggaattgta ctgtttgccc cgcaacttga taaccttctg 360

aaaaaaaacc cgaaaattgg aaatacattg ggttcggcaa gctctatcag tcagaacatt 420

ggcaaagcta acactgtgtt aggtggtatt cagtctattc tggggtcggt cttgtccggc 480

gttaacctga atgaactgct tcaaaacaaa gatccaaatc aactggaact ggccaaagcg 540

ggcctggagt taacgaacga gttggtagga aacattgcct cctctgtgca gacggtggat 600

gcatttgctg aacagattag caaattaggt tctcatctgc aaaacgtcaa aggactgggc 660

ggcctcagca acaaactgca gaatttaccg gatctgggca aagccagcct tgggttagat 720

attatctccg ggctcttgtc aggcgcgtca gccggtctta tcctggcgga caaagaagca 780

agcaccgaaa agaaagctgc cgcaggcgtc gagtttgcga accagattat tggaaatgtc 840

accaaagcgg tcagttccta catcctggcg cagcgtgtag cgtcgggcct gtcatctact 900

ggtccagttg ccgcgttgat tgcgtctacg gtcgccttag cggtttcgcc gctgtccttt 960

ctgaacgtgg cggataagtt caaacaagcg gatttgatca aaagctatag tgaacgcttt 1020

cagaaattag gttatgatgg tgaccgtctg ctggcggatt tccaccgcga aaccggaacc 1080

attgatgcct ctgtcactac catcaatacg gctttggcag cgatctccgg tggggtgggc 1140

gcggcgagcg ctggctcact ggtaggagcc cctgttgctc ttttggttgc aggcgttaca 1200

ggcctgatta cgaccatttt agagtatagc aaacaagcga tgtttgagca cgtcgcaaat 1260

aaggtacacg atcgtattgt agagtgggaa aagaaacata acaagaatta cttcgaacag 1320

gggtatgatt cacgccatct ggcagatctt caggacaaca tgaaatttct gatcaatctg 1380

aacaaagagc tgcaagcaga acgcgttgtg gcgattacac aacagcgttg ggataaccag 1440

attggcgatc tggcagcaat ctcgcgccgt acggacaaaa tttcttcggg taaagcctac 1500

gtggacgcct ttgaagaagg acagcatcag tcttatgatt cctcggtaca gctggacaac 1560

aaaaatggaa tcatcaacat tagtaatacc aaccgcaaga cccaaagtgt cctgtttcgc 1620

actcccctgc tcaccccggg cgaagagaac cgcgaacgca ttcaagaggg tgcaaactca 1680

tacattacca aattgcacat tcagcgtgtg gatagttgga ctgtcactga cggtgatgcg 1740

agcagcagtg ttgactttac taacgtggtt caacgcattg ccgtcaaatt cgacgacgct 1800

gggaatatta ttgaaagcaa agatacgaaa attatcgcta atttgggcgc tggtaatgat 1860

aatgtgttcg tgggttccag caccacggtt attgatggtg gcgatgggca tgaccgcgtg 1920

cattactcgc gtggcgaata tggcgccctt gtaatcgacg ccaccgcgga aacagagaaa 1980

ggtagctatt cggtgaaacg ctatgtgggt gattctaaag cgttacacga aaccattgcc 2040

acacatcaga ccaacgtggg cgctcgtgaa gagaaaatcg aatatcggcg tgaagatgat 2100

cgttttcata caggctacac cgtaacggac tctctcaaaa gcgttgaaga aattatcggc 2160

agccagttca atgatatctt caaaggctca caattcgacg atgtgttcca tggaggtaat 2220

ggggttgaca ccattgatgg caatgacggc gatgatcatc tgtttggcgg cgctggtgac 2280

gatgtgatcg atggtggtaa cggtaataat tttctcgtcg gcggtaccgg gaacgatatt 2340

atcagcggcg gtaaggacaa tgatatttat gtgcacaaga ctggtgatgg caacgactcc 2400

atcaccgata gcggtggcca ggataaactg gcatttagcg atgtgaacct caaagatctg 2460

acctttaaaa aagtggactc tagtctggaa atcattaacc agaagggcga gaaagtccgc 2520

attggtaact ggtttctgga agatgactta gctagcaccg tggcgaacta taaagccacg 2580

aatgatcgca aaattgaaga aatcatcggc aagggcggcg agcgtattac ttcggaacag 2640

gtggataaac tgatcaaaga gggtaacaat caaattagcg ccgaagcgct gtcgaaagta 2700

gtgaacgact acaatacgag taaagatcgc cagaatgttt ccaactcgct ggccaagctg 2760

atctccagtg ttggctcatt caccagttcg agtgatttcc ggaacaatct cggtacgtat 2820

gttccttctt ccatcgacgt tagtaacaat attcagttgg cacgcgcagc ataagagctc 2880

<210> 12
<211> 3168
<212> DNA
<213> Actonbacillus pleuropneumoniae

<220>
<223> ApxIIIA K571A K702A

<400> 12
ggatccagca catggagcag tatgcttgcg gacttgaaga aacgggcgga agaagcgaaa 60

cgccaagcga agaaaggata tgatgtgact aagaacggcc tgcagtacgg tgtatcgcaa 120

gctaaactgc aggccctggc ggccgggaaa gcggttcaga agtatggtaa caaactcgtc 180

cttgtgattc ccaaagaata cgatggcagc gtgggtaatg gtttctttga tctggttaaa 240

gcggccgagg aactcggcat tcaggttaaa tatgtcaatc gtaacgagct tgaggttgcg 300

cacaaatccc tgggtactgc ggatcaattc ctcggcctca cggaacgtgg tctgactctg 360

ttcgccccac agctcgatca gttcttacag aagcacagca agatttccaa tgtagtcggg 420

agtagcactg gtgatgctgt cagcaaactt gctaaatcgc agactatcat ctcgggtatt 480

cagtcagtgc tgggcacggt tctggcgggc atcaacctga acgaagctat tatcagtggt 540

ggttccgaat tagaactggc cgaagccggg gtgagtcttg cctcagaact ggtgagcaat 600

attgcgaaag gcacgaccac gattgatgct ttcaccaccc aaatccagaa ttttgggaaa 660

ttggtggaaa atgcgaaagg cctgggaggc gtaggccgcc agctgcaaaa catttcgggg 720

agcgccctct ctaaaaccgg cctggggtta gacattatct cttcgctgct gtcaggagtt 780

accgcgagct ttgccctggc aaacaagaat gccagcacgt ctactaaagt ggcagcgggt 840

tttgaactct caaatcaggt cattggtggc attactaaag ccgtatcgag ttacattctg 900

gcgcaacgtc tggctgcagg cctgtctacg accggccctg cggcagcgct gattgcgtct 960

tctatctcgt tggcgatttc gcctctggcg tttctgcgtg ttgctgataa tttcaatcgg 1020

tccaaagaaa tcggggagtt tgcagagcgt tttaagaagc tgggatatga cggtgataaa 1080

ttgctgagcg aattttacca tgaggcgggc actatcgacg cctccattac caccatttca 1140

accgcactga gcgcgatcgc agcaggaacg gcggcagctt cggcgggtgc attagtggga 1200

gcaccgatta ccctgttggt taccgggatt accgggttaa ttagcggcat tctggaattc 1260

agtaaacaac cgatgctgga tcatgtcgca agcaaaatcg gtaacaaaat tgacgaatgg 1320

gaaaaaaaat acggcaaaaa ctacttcgaa aatggctatg acgcgcgcca caaggccttt 1380

ctcgaagatt cattttcact gttgagcagc ttcaataagc agtatgaaac ggaacgtgcc 1440

gttttgatca cgcagcagcg ttgggatgaa tacattggcg aattagccgg aattacgggc 1500

aaaggtgaca aactgtcctc tggcaaagcc tatgtggact acttccaaga aggtaaactg 1560

ctcgaaaaaa aaccagatga tttctcgaaa gtggtctttg atcccacgaa gggtgagatc 1620

gacatcagca attctcagac atctaccctg ctgaagtttg ttacgccgtt gttgaccccg 1680

ggtacggaat cacgcgaacg cacacagaca ggcgcctatg agtacattac caaattagtg 1740

gtaaaaggca aagataaatg ggtagttaac ggcgtgaaag acaaaggagc agtgtatgac 1800

tacacgaact taattcagca tgcacacatt agctcctccg tcgcacgcgg tgaagagtac 1860

cgtgaagttc gccttgtaag tcatttgggt aacggaaatg ataaagtgtt cctcgccgca 1920

ggaagtgcgg aaattcatgc tggcgaaggt catgacgtcg tgtactatga taaaaccgat 1980

acaggccttc tggttattga cggcactaaa gctaccgaac aggggcgtta cagtgtgacc 2040

cgcgaacttt ccggtgctac caaaatctta cgcgaagtca tcaagaatca gaaaagtgcc 2100

gtaggcgcgc gtgaagagac actggaatat cgggactatg agttaacgca aagcgggaac 2160

agtaatttaa aggctcacga tgaactgcat tccgtagagg agattatcgg cagcaaccag 2220

cgtgatgaat ttaaaggcag caaattccgc gatatcttcc atggtgccga cggtgacgac 2280

cttcttaacg ggaatgatgg ggatgacatc ctgtatggtg acaagggcaa tgatgaactg 2340

cgtggcgata acggcaatga ccagctgtac ggtggtgagg ggaatgacaa attgttaggt 2400

ggcaacggaa acaactatct gtccggcggc gacggtaatg atgagttgca agttcttggc 2460

aatggcttta acgtgctgcg cggcggcaaa ggtgatgata aactgtatgg tagttcaggc 2520

tcagatttgc tggatggcgg ggaaggtaac gattatctgg aaggcggtga tggctctgat 2580

ttctatgtgt atcgctcgac ctctggcaac cataccattt atgaccaggg aaaatcctcg 2640

gatctggata aactctatct gtccgatttt agttttgatc gcctgctggt cgagaaagtt 2700

gatgacaatc tggttctgcg ctcgaacgaa tctagccaca acaatggtgt gcttaccatc 2760

aaagattggt ttaaagaggg taacaaatac aatcacaaaa tcgagcaaat tgtggataag 2820

aatggtcgca agctgaccgc cgagaattta gggacttact ttaaaaatgc cccgaaagcg 2880

gataatctcc tgaactatgc aaccaaggag gaccagaacg aatcgaacct gtcctctctg 2940

aaaacggaac tgagcaaaat cattaccaac gcaggcaact ttggcgtggc taaacaaggg 3000

aacacaggga tcaataccgc ggcgctgaac aacgaagtca acaaaattat cagtagcgcc 3060

aacacatttg cgacctctca actgggtggt agcggtatgg gaaccttgcc gtcaacgaat 3120

gtcaacagca tgatgctggg caacttagcc cgcgctgcat aagagctc 3168

<210> 13
<211> 527
<212> DNA
<213> Actonbacillus pleuropneumoniae

<220>
<223> ApxIC

<400> 13
   tcatgagtaa aaaaattaat ggatttgagg ttttaggaga ggtggcatgg ttatgggcaa 60
   gttctccttt acatcgaaag tggccgcttt ctttgttagc aattaatgtg ctacctgcga 120
   ttgagagtaa tcaatatgtt ttgttaaagc gtgacggttt tcctattgca ttttgtagct 180
   gggcaaattt gaatttggaa aatgaaatta aataccttga tgatgttgcc tcgctagttg 240
   cggatgattg gacttccggc gatcgtcgat ggtttataga ttggatagca ccgttcggag 300
   acagtgccgc attatacaaa catatgcgag ataacttccc gaatgagctg tttagggcta 360
   ttcgagttga tccggactct cgagtaggga aaatttcaga atttcatgga ggaaaaattg 420
   ataagaaact ggcaagtaaa atttttcaac aatatcactt tgaattaatg agtgagctaa 480
   aaaataaaca aaattttaaa ttttcattag taaatagcta aagatct 527

<210> 14
<211> 488
<212> DNA
<213> Actonbacillus pleuropneumoniae

<220>
<223> ApxIIC

<400> 14
acatgttaaa aaatgatttt aacgtattgg gacaaattgc ttggttatgg gcaaattctc 60

caatgcaccg aaattggtca gtttcactgt taatgaagaa tgttattcct gcaattgaaa 120

atgaccaata tttgttacta gttgatgatg gttttcctat tgcatattgc agttgggcca 180

aattaactct agagagtgag gctcgctatg taaaggacac caattcatta aaaatagatg 240

attggaatgc aggagatcgt atatggatca ttgattggat tgccccattc ggggattcat 300

ctctattgta taaacatatg agacaacgtt ttccatacga tattggaagg gcaattagaa 360

tctatcctag caaaaaagat actggaaaaa tcatatattt aaaaggagga aaaataacaa 420

aaaaagtagc tgaaaagaca tttcttcagt atgagcaaga gttaataaca gctctacaat 480

aaagatct 488

<210> 15

<211> 530
<212> DNA
<213> Actonbacillus pleuropneumoniae

<220>
<223> ApxIIIC

<400> 15
tcatgagtta taaaaatgtt aaaaatttaa cagatgattt tacaacttta gggcatatcg 60

cttggttgtg ggctaattct ccgttacata aggagtggtc tatctctttg tttactaaga 120

atattttgcc agccattcaa catgatcaat atattttact tatgcgagat gagttccctg 180

tagcgttttg tagttgggca aatttaacgt taactaatga agtgaagtat gtacgtgatg 240

tgacgtcatt gacttttgaa gattggaatt caggagaacg aaaatggttg atcgactgga 300

ttgcgccatt tggggataac aatacgcttt atagatatat gcgtaaaaaa tttcctaatg 360

aagtattccg ggccattcga gtatatcctg gttctacaga agcgaaaatc attcatgttc 420

aaggaggaca aattaataaa tttacagcta aaaaattaat acaacaatat caggaagaac 480

ttattcaagt tcttaacaat cacaaaaaaa ttgtaagagg ataaagatct 530

<210> 16
<211> 639
<212> DNA
<213> Actonbacillus pleuropneumoniae

<220>
<223> ApxIC with promoter/lac-operator and RBS

<400> 16
tctagaaaat cataaaaaat ttatttgctt tgtgagcgga taacaattat aatagattca 60

attgtgagcg gataacaatt tcacacagaa ttcattaaag aggagaaatt aaccatgagt 120

aaaaaaatta atggatttga ggttttagga gaggtggcat ggttatgggc aagttctcct 180

ttacatcgaa agtggccgct ttctttgtta gcaattaatg tgctacctgc gattgagagt 240

aatcaatatg ttttgttaaa gcgtgacggt tttcctattg cattttgtag ctgggcaaat 300

ttgaatttgg aaaatgaaat taaatacctt gatgatgttg cctcgctagt tgcggatgat 360

tggacttccg gcgatcgtcg atggtttata gattggatag caccgttcgg agacagtgcc 420

gcattataca aacatatgcg agataacttc ccgaatgagc tgtttagggc tattcgagtt 480

gatccggact ctcgagtagg gaaaatttca gaatttcatg gaggaaaaat tgataagaaa 540

ctggcaagta aaatttttca acaatatcac tttgaattaa tgagtgagct aaaaaataaa 600

caaaatttta aattttcatt agtaaatagc taatcgcga 639

<210> 17
<211> 600

<212> DNA
<213> Actonbacillus pleuropneumoniae

<220>
<223> ApxIIC with promoter/lac-operator and RBS

<400> 17
atcgataaat cataaaaaat ttatttgctt tgtgagcgga taacaattat aatagattca 60

attgtgagcg gataacaatt tcacacagaa ttcattaaag aggagaaatt aaccatgtta 120

aaaaatgatt ttaacgtatt gggacaaatt gcttggttat gggcaaattc tccaatgcac 180

cgaaattggt cagtttcact gttaatgaag aatgttattc ctgcaattga aaatgaccaa 240

tatttgttac tagttgatga tggttttcct attgcatatt gcagttgggc caaattaact 300

ctagagagtg aggctcgcta tgtaaaggac accaattcat taaaaataga tgattggaat 360

gcaggagatc gtatatggat cattgattgg attgccccat tcggggattc atctctattg 420

tataaacata tgagacaacg ttttccatac gatattggaa gggcaattag aatctatcct 480

agcaaaaaag atactggaaa aatcatatat ttaaaaggag gaaaaataac aaaaaaagta 540

gctgaaaaga catttcttca gtatgagcaa gagttaataa cagctctaca ataatcgcga 600

<210> 18
<211> 642
<212> DNA
<213> Actonbacillus pleuropneumoniae

<220>
<223> ApxIIIC with promoter/lac-operator and RBS

<400> 18
tctagaaaat cataaaaaat ttatttgctt tgtgagcgga taacaattat aatagattca 60

attgtgagcg gataacaatt tcacacagaa ttcattaaag aggagaaatt aaccatgagt 120

tataaaaatg ttaaaaattt aacagatgat tttacaactt tagggcatat cgcttggttg 180

tgggctaatt ctccgttaca taaggagtgg tctatctctt tgtttactaa gaatattttg 240

ccagccattc aacatgatca atatatttta cttatgcgag atgagttccc tgtagcgttt 300

tgtagttggg caaatttaac gttaactaat gaagtgaagt atgtacgtga tgtgacgtca 360

ttgacttttg aagattggaa ttcaggagaa cgaaaatggt tgatcgattg gattgcgcca 420

tttggggata acaatacgct ttatagatat atgcgtaaaa aatttcctaa tgaagtattc 480

cgggccattc gagtatatcc tggttctaca gaagcgaaaa tcattcatgt tcaaggagga 540

caaattaata aatttacagc taaaaaatta atacaacaat atcaggaaga acttattcaa 600

gttcttaaca atcacaaaaa aattgtaaga ggataatcgc ga 642

<210> 19

<211> 2446
<212> DNA
<213> Plasmid pEX-A258

<220>
<223> pEX-A258

<400> 19
gtggcagctc tagagctagc gaattctttg gtgaaattgt tatccgctca caattccaca 60

caacatacga gccggaagca taaagtgtaa agcctggggt gcctaatgag tgagctaact 120

cacattaatt gcgttgcgct cactgcccgc tttccagtcg ggaaacctgt cgtgccagct 180

gcattaatga atcggccaac gcgcggggag aggcggtttg cgtattgggc gctcttccgc 240

ttcctcgctc actgactcgc tgcgctcggt cgttcggctg cggcgagcgg tatcagctca 300

ctcaaaggcg gtaatacggt tatccacaga atcaggggat aacgcaggaa agaacatgtg 360

agcaaaaggc cagcaaaagg ccaggaaccg taaaaaggcc gcgttgctgg cgtttttcca 420

taggctccgc ccccctgacg agcatcacaa aaatcgacgc tcaagtcaga ggtggcgaaa 480

cccgacagga ctataaagat accaggcgtt tccccctgga agctccctcg tgcgctctcc 540

tgttccgacc ctgccgctta ccggatacct gtccgccttt ctcccttcgg gaagcgtggc 600

gctttctcat agctcacgct gtaggtatct cagttcggtg taggtcgttc gctccaagct 660

gggctgtgtg cacgaacccc ccgttcagcc cgaccgctgc gccttatccg gtaactatcg 720

tcttgagtcc aacccggtaa gacacgactt atcgccactg gcagcagcca ctggtaacag 780

gattagcaga gcgaggtatg taggcggtgc tacagagttc ttgaagtggt ggcctaacta 840

cggctacact agaagaacag tatttggtat ctgcgctctg ctgaagccag ttaccttcgg 900

aaaaagagtt ggtagctctt gatccggcaa acaaaccacc gctggtagcg gtggtttttt 960

tgtttgcaag cagcagatta cgcgcagaaa aaaaggatct caagaagatc ctttgatctt 1020

ttctacgggg tctgacgctc agtggaacga aaactcacgt taagggattt tggtcatgag 1080

attatcaaaa aggatcttca cctagatcct tttaaattaa aaatgaagtt ttaaatcaat 1140

ctaaagtata tatgagtaaa cttggtctga cagttaccaa tgcttaatca gtgaggcacc 1200

tatctcagcg atctgtctat ttcgttcatc catagttgcc tgactccccg tcgtgtagat 1260

aactacgata cgggagggct taccatctgg ccccagtgct gcaatgatac cgcgcgaacc 1320

acgctcaccg gctccagatt tatcagcaat aaaccagcca gccggaaggg ccgagcgcag 1380

aagtggtcct gcaactttat ccgcctccat ccagtctatt aattgttgcc gggaagctag 1440

agtaagtagt tcgccagtta atagtttgcg caacgttgtt gccattgcta caggcatcgt 1500

ggtgtcacgc tcgtcgtttg gtatggcttc attcagctcc ggttcccaac gatcaaggcg 1560

agttacatga tcccccatgt tgtgcaaaaa agcggttagc tccttcggtc ctccgatcgt 1620

tgtcagaagt aagttggccg cagtgttatc actcatggtt atggcagcac tgcataattc 1680

tcttactgtc atgccatccg taagatgctt ttctgtgact ggtgagtact caaccaagtc 1740

attctgagaa tagtgtatgc ggcgaccgag ttgctcttgc ccggcgtcaa tacgggataa 1800

taccgcgcca catagcagaa ctttaaaagt gctcatcatt ggaaaacgtt cttcggggcg 1860

aaaactctca aggatcttac cgctgttgag atccagttcg atgtaaccca ctcgtgcacc 1920

caactgatct tcagcatctt ttactttcac cagcgtttct gggtgagcaa aaacaggaag 1980

gcaaaatgcc gcaaaaaagg gaataagggc gacacggaaa tgttgaatac tcatactctt 2040

cctttttcaa tattattgaa gcatttatca gggttattgt ctcatgagcg gatacatatt 2100

tgaatgtatt tagaaaaata aacaaatagg ggttccgcgc acatttcccc gaaaagtgcc 2160

acctgacgtc taagaaacca ttattatcat gacattaacc tataaaaata ggcgtatcac 2220

gaggcccttt cgtctcgcgc gtttcggtga tgacggtgaa aacctctgac acatgcagct 2280

cccggagacg gtcacagctt gtctgtaagc ggatgccggg agcagacaag cccgtcaggg 2340

cgcgtcagcg ggtgttggcg ggtgtcgggg ctggcttaac tatgcggcat cagagcagat 2400

tgtactgaga gtttggcaat tggtcgacct cgagggcgcg cccgta 2446

<210> 20
<211> 4751
<212> DNA
<213> Plasmid pQE-80L

<220>
<223> pQE-80L

<400> 20
ctcgagaaat cataaaaaat ttatttgctt tgtgagcgga taacaattat aatagattca 60

attgtgagcg gataacaatt tcacacagaa ttcattaaag aggagaaatt aactatgaga 120

ggatcgcatc accatcacca tcacggatcc gcatgcgagc tcggtacccc gggtcgacct 180

gcagccaagc ttaattagct gagcttggac tcctgttgat agatccagta atgacctcag 240

aactccatct ggatttgttc agaacgctcg gttgccgccg ggcgtttttt attggtgaga 300

atccaagcta gcttggcgag attttcagga gctaaggaag ctaaaatgga gaaaaaaatc 360

actggatata ccaccgttga tatatcccaa tggcatcgta aagaacattt tgaggcattt 420

cagtcagttg ctcaatgtac ctataaccag accgttcagc tggatattac ggccttttta 480

aagaccgtaa agaaaaataa gcacaagttt tatccggcct ttattcacat tcttgcccgc 540

ctgatgaatg ctcatccgga atttcgtatg gcaatgaaag acggtgagct ggtgatatgg 600

gatagtgttc acccttgtta caccgttttc catgagcaaa ctgaaacgtt ttcatcgctc 660

tggagtgaat accacgacga tttccggcag tttctacaca tatattcgca agatgtggcg 720

tgttacggtg aaaacctggc ctatttccct aaagggttta ttgagaatat gtttttcgtc 780

tcagccaatc cctgggtgag tttcaccagt tttgatttaa acgtggccaa tatggacaac 840

ttcttcgccc ccgttttcac catgggcaaa tattatacgc aaggcgacaa ggtgctgatg 900

ccgctggcga ttcaggttca tcatgccgtt tgtgatggct tccatgtcgg cagaatgctt 960

aatgaattac aacagtactg cgatgagtgg cagggcgggg cgtaattttt ttaaggcagt 1020

tattggtgcc cttaaacgcc tggggtaatg actctctagc ttgaggcatc aaataaaacg 1080

aaaggctcag tcgaaagact gggcctttcg ttttatctgt tgtttgtcgg tgaacgctct 1140

cctgagtagg acaaatccgc cctctagatt acgtgcagtc gatgataagc tgtcaaacat 1200

gagaattgtg cctaatgagt gagctaactt acattaattg cgttgcgctc actgcccgct 1260

ttccagtcgg gaaacctgtc gtgccagctg cattaatgaa tcggccaacg cgcggggaga 1320

ggcggtttgc gtattgggcg ccagggtggt ttttcttttc accagtgaga cgggcaacag 1380

ctgattgccc ttcaccgcct ggccctgaga gagttgcagc aagcggtcca cgctggtttg 1440

ccccagcagg cgaaaatcct gtttgatggt ggttaacggc gggatataac atgagctgtc 1500

ttcggtatcg tcgtatccca ctaccgagat atccgcacca acgcgcagcc cggactcggt 1560

aatggcgcgc attgcgccca gcgccatctg atcgttggca accagcatcg cagtgggaac 1620

gatgccctca ttcagcattt gcatggtttg ttgaaaaccg gacatggcac tccagtcgcc 1680

ttcccgttcc gctatcggct gaatttgatt gcgagtgaga tatttatgcc agccagccag 1740

acgcagacgc gccgagacag aacttaatgg gcccgctaac agcgcgattt gctggtgacc 1800

caatgcgacc agatgctcca cgcccagtcg cgtaccgtct tcatgggaga aaataatact 1860

gttgatgggt gtctggtcag agacatcaag aaataacgcc ggaacattag tgcaggcagc 1920

ttccacagca atggcatcct ggtcatccag cggatagtta atgatcagcc cactgacgcg 1980

ttgcgcgaga agattgtgca ccgccgcttt acaggcttcg acgccgcttc gttctaccat 2040

cgacaccacc acgctggcac ccagttgatc ggcgcgagat ttaatcgccg cgacaatttg 2100

cgacggcgcg tgcagggcca gactggaggt ggcaacgcca atcagcaacg actgtttgcc 2160

cgccagttgt tgtgccacgc ggttgggaat gtaattcagc tccgccatcg ccgcttccac 2220

tttttcccgc gttttcgcag aaacgtggct ggcctggttc accacgcggg aaacggtctg 2280

ataagagaca ccggcatact ctgcgacatc gtataacgtt actggtttca cattcaccac 2340

cctgaattga ctctcttccg ggcgctatca tgccataccg cgaaaggttt tgcaccattc 2400

gatggtgtcg gaatttcggg cagcgttggg tcctggccac gggtgcgcat gatctagagc 2460

tgcctcgcgc gtttcggtga tgacggtgaa aacctctgac acatgcagct cccggagacg 2520

gtcacagctt gtctgtaagc ggatgccggg agcagacaag cccgtcaggg cgcgtcagcg 2580

ggtgttggcg ggtgtcgggg cgcagccatg acccagtcac gtagcgatag cggagtgtat 2640

actggcttaa ctatgcggca tcagagcaga ttgtactgag agtgcaccat atgcggtgtg 2700

aaataccgca cagatgcgta aggagaaaat accgcatcag gcgctcttcc gcttcctcgc 2760

tcactgactc gctgcgctcg gtcgttcggc tgcggcgagc ggtatcagct cactcaaagg 2820

cggtaatacg gttatccaca gaatcagggg ataacgcagg aaagaacatg tgagcaaaag 2880

gccagcaaaa ggccaggaac cgtaaaaagg ccgcgttgct ggcgtttttc cataggctcc 2940

gcccccctga cgagcatcac aaaaatcgac gctcaagtca gaggtggcga aacccgacag 3000

gactataaag ataccaggcg tttccccctg gaagctccct cgtgcgctct cctgttccga 3060

ccctgccgct taccggatac ctgtccgcct ttctcccttc gggaagcgtg gcgctttctc 3120

atagctcacg ctgtaggtat ctcagttcgg tgtaggtcgt tcgctccaag ctgggctgtg 3180

tgcacgaacc ccccgttcag cccgaccgct gcgccttatc cggtaactat cgtcttgagt 3240

ccaacccggt aagacacgac ttatcgccac tggcagcagc cactggtaac aggattagca 3300

gagcgaggta tgtaggcggt gctacagagt tcttgaagtg gtggcctaac tacggctaca 3360

ctagaaggac agtatttggt atctgcgctc tgctgaagcc agttaccttc ggaaaaagag 3420

ttggtagctc ttgatccggc aaacaaacca ccgctggtag cggtggtttt tttgtttgca 3480

agcagcagat tacgcgcaga aaaaaaggat ctcaagaaga tcctttgatc ttttctacgg 3540

ggtctgacgc tcagtggaac gaaaactcac gttaagggat tttggtcatg agattatcaa 3600

aaaggatctt cacctagatc cttttaaatt aaaaatgaag ttttaaatca atctaaagta 3660

tatatgagta aacttggtct gacagttacc aatgcttaat cagtgaggca cctatctcag 3720

cgatctgtct atttcgttca tccatagttg cctgactccc cgtcgtgtag ataactacga 3780

tacgggaggg cttaccatct ggccccagtg ctgcaatgat accgcgagac ccacgctcac 3840

cggctccaga tttatcagca ataaaccagc cagccggaag ggccgagcgc agaagtggtc 3900

ctgcaacttt atccgcctcc atccagtcta ttaattgttg ccgggaagct agagtaagta 3960

gttcgccagt taatagtttg cgcaacgttg ttgccattgc tacaggcatc gtggtgtcac 4020

gctcgtcgtt tggtatggct tcattcagct ccggttccca acgatcaagg cgagttacat 4080

gatcccccat gttgtgcaaa aaagcggtta gctccttcgg tcctccgatc gttgtcagaa 4140

gtaagttggc cgcagtgtta tcactcatgg ttatggcagc actgcataat tctcttactg 4200

tcatgccatc cgtaagatgc ttttctgtga ctggtgagta ctcaaccaag tcattctgag 4260

aatagtgtat gcggcgaccg agttgctctt gcccggcgtc aatacgggat aataccgcgc 4320

cacatagcag aactttaaaa gtgctcatca ttggaaaacg ttcttcgggg cgaaaactct 4380

caaggatctt accgctgttg agatccagtt cgatgtaacc cactcgtgca cccaactgat 4440

cttcagcatc ttttactttc accagcgttt ctgggtgagc aaaaacagga aggcaaaatg 4500

ccgcaaaaaa gggaataagg gcgacacgga aatgttgaat actcatactc ttcctttttc 4560

aatattattg aagcatttat cagggttatt gtctcatgag cggatacata tttgaatgta 4620

tttagaaaaa taaacaaata ggggttccgc gcacatttcc ccgaaaagtg ccacctgacg 4680

tctaagaaac cattattatc atgacattaa cctataaaaa taggcgtatc acgaggccct 4740

ttcgtcttca c 4751

<210> 21
<211> 3431
<212> DNA
<213> Plasmid pQE-60

<220>
<223> pQE-60

<400> 21
ctcgagaaat cataaaaaat ttatttgctt tgtgagcgga taacaattat aatagattca 60

attgtgagcg gataacaatt tcacacagaa ttcattaaag aggagaaatt aaccatggga 120

ggatccagat ctcatcacca tcaccatcac taagcttaat tagctgagct tggactcctg 180

ttgatagatc cagtaatgac ctcagaactc catctggatt tgttcagaac gctcggttgc 240

cgccgggcgt tttttattgg tgagaatcca agctagcttg gcgagatttt caggagctaa 300

ggaagctaaa atggagaaaa aaatcactgg atataccacc gttgatatat cccaatggca 360

tcgtaaagaa cattttgagg catttcagtc agttgctcaa tgtacctata accagaccgt 420

tcagctggat attacggcct ttttaaagac cgtaaagaaa aataagcaca agttttatcc 480

ggcctttatt cacattcttg cccgcctgat gaatgctcat ccggaatttc gtatggcaat 540

gaaagacggt gagctggtga tatgggatag tgttcaccct tgttacaccg ttttccatga 600

gcaaactgaa acgttttcat cgctctggag tgaataccac gacgatttcc ggcagtttct 660

acacatatat tcgcaagatg tggcgtgtta cggtgaaaac ctggcctatt tccctaaagg 720

gtttattgag aatatgtttt tcgtctcagc caatccctgg gtgagtttca ccagttttga 780

tttaaacgtg gccaatatgg acaacttctt cgcccccgtt ttcaccatgc atgggcaaat 840

attatacgca aggcgacaag gtgctgatgc cgctggcgat tcaggttcat catgccgtct 900

gtgatggctt ccatgtcggc agaatgctta atgaattaca acagtactgc gatgagtggc 960

agggcggggc gtaatttttt taaggcagtt attggtgccc ttaaacgcct ggggtaatga 1020

ctctctagct tgaggcatca aataaaacga aaggctcagt cgaaagactg ggcctttcgt 1080

tttatctgtt gtttgtcggt gaacgctctc ctgagtagga caaatccgcc gctctagagc 1140

tgcctcgcgc gtttcggtga tgacggtgaa aacctctgac acatgcagct cccggagacg 1200

gtcacagctt gtctgtaagc ggatgccggg agcagacaag cccgtcaggg cgcgtcagcg 1260

ggtgttggcg ggtgtcgggg cgcagccatg acccagtcac gtagcgatag cggagtgtat 1320

actggcttaa ctatgcggca tcagagcaga ttgtactgag agtgcaccat atgcggtgtg 1380

aaataccgca cagatgcgta aggagaaaat accgcatcag gcgctcttcc gcttcctcgc 1440

tcactgactc gctgcgctcg gtcgttcggc tgcggcgagc ggtatcagct cactcaaagg 1500

cggtaatacg gttatccaca gaatcagggg ataacgcagg aaagaacatg tgagcaaaag 1560

gccagcaaaa ggccaggaac cgtaaaaagg ccgcgttgct ggcgtttttc cataggctcc 1620

gcccccctga cgagcatcac aaaaatcgac gctcaagtca gaggtggcga aacccgacag 1680

gactataaag ataccaggcg tttccccctg gaagctccct cgtgcgctct cctgttccga 1740

ccctgccgct taccggatac ctgtccgcct ttctcccttc gggaagcgtg gcgctttctc 1800

atagctcacg ctgtaggtat ctcagttcgg tgtaggtcgt tcgctccaag ctgggctgtg 1860

tgcacgaacc ccccgttcag cccgaccgct gcgccttatc cggtaactat cgtcttgagt 1920

ccaacccggt aagacacgac ttatcgccac tggcagcagc cactggtaac aggattagca 1980

gagcgaggta tgtaggcggt gctacagagt tcttgaagtg gtggcctaac tacggctaca 2040

ctagaaggac agtatttggt atctgcgctc tgctgaagcc agttaccttc ggaaaaagag 2100

ttggtagctc ttgatccggc aaacaaacca ccgctggtag cggtggtttt tttgtttgca 2160

agcagcagat tacgcgcaga aaaaaaggat ctcaagaaga tcctttgatc ttttctacgg 2220

ggtctgacgc tcagtggaac gaaaactcac gttaagggat tttggtcatg agattatcaa 2280

aaaggatctt cacctagatc cttttaaatt aaaaatgaag ttttaaatca atctaaagta 2340

tatatgagta aacttggtct gacagttacc aatgcttaat cagtgaggca cctatctcag 2400

cgatctgtct atttcgttca tccatagttg cctgactccc cgtcgtgtag ataactacga 2460

tacgggaggg cttaccatct ggccccagtg ctgcaatgat accgcgagac ccacgctcac 2520

cggctccaga tttatcagca ataaaccagc cagccggaag ggccgagcgc agaagtggtc 2580

ctgcaacttt atccgcctcc atccagtcta ttaattgttg ccgggaagct agagtaagta 2640

gttcgccagt taatagtttg cgcaacgttg ttgccattgc tacaggcatc gtggtgtcac 2700

gctcgtcgtt tggtatggct tcattcagct ccggttccca acgatcaagg cgagttacat 2760

gatcccccat gttgtgcaaa aaagcggtta gctccttcgg tcctccgatc gttgtcagaa 2820

gtaagttggc cgcagtgtta tcactcatgg ttatggcagc actgcataat tctcttactg 2880

tcatgccatc cgtaagatgc ttttctgtga ctggtgagta ctcaaccaag tcattctgag 2940

aatagtgtat gcggcgaccg agttgctctt gcccggcgtc aatacgggat aataccgcgc 3000

cacatagcag aactttaaaa gtgctcatca ttggaaaacg ttcttcgggg cgaaaactct 3060

caaggatctt accgctgttg agatccagtt cgatgtaacc cactcgtgca cccaactgat 3120

cttcagcatc ttttactttc accagcgttt ctgggtgagc aaaaacagga aggcaaaatg 3180

ccgcaaaaaa gggaataagg gcgacacgga aatgttgaat actcatactc ttcctttttc 3240

aatattattg aagcatttat cagggttatt gtctcatgag cggatacata tttgaatgta 3300

tttagaaaaa taaacaaata ggggttccgc gcacatttcc ccgaaaagtg ccacctgacg 3360

tctaagaaac cattattatc atgacattaa cctataaaaa taggcgtatc acgaggccct 3420

ttcgtcttca c 3431

<210> 22
<211> 4245
<212> DNA
<213> Plasmid pACYC184

<220>
<223> pACYC184

<400> 22
gaattccgga tgagcattca tcaggcgggc aagaatgtga ataaaggccg gataaaactt 60

gtgcttattt ttctttacgg tctttaaaaa ggccgtaata tccagctgaa cggtctggtt 120

ataggtacat tgagcaactg actgaaatgc ctcaaaatgt tctttacgat gccattggga 180

tatatcaacg gtggtatatc cagtgatttt tttctccatt ttagcttcct tagctcctga 240

aaatctcgat aactcaaaaa atacgcccgg tagtgatctt atttcattat ggtgaaagtt 300

ggaacctctt acgtgccgat caacgtctca ttttcgccaa aagttggccc agggcttccc 360

ggtatcaaca gggacaccag gatttattta ttctgcgaag tgatcttccg tcacaggtat 420

ttattcggcg caaagtgcgt cgggtgatgc tgccaactta ctgatttagt gtatgatggt 480

gtttttgagg tgctccagtg gcttctgttt ctatcagctg tccctcctgt tcagctactg 540

acggggtggt gcgtaacggc aaaagcaccg ccggacatca gcgctagcgg agtgtatact 600

ggcttactat gttggcactg atgagggtgt cagtgaagtg cttcatgtgg caggagaaaa 660

aaggctgcac cggtgcgtca gcagaatatg tgatacagga tatattccgc ttcctcgctc 720

actgactcgc tacgctcggt cgttcgactg cggcgagcgg aaatggctta cgaacggggc 780

ggagatttcc tggaagatgc caggaagata cttaacaggg aagtgagagg gccgcggcaa 840

agccgttttt ccataggctc cgcccccctg acaagcatca cgaaatctga cgctcaaatc 900

agtggtggcg aaacccgaca ggactataaa gataccaggc gtttccccct ggcggctccc 960

tcgtgcgctc tcctgttcct gcctttcggt ttaccggtgt cattccgctg ttatggccgc 1020

gtttgtctca ttccacgcct gacactcagt tccgggtagg cagttcgctc caagctggac 1080

tgtatgcacg aaccccccgt tcagtccgac cgctgcgcct tatccggtaa ctatcgtctt 1140

gagtccaacc cggaaagaca tgcaaaagca ccactggcag cagccactgg taattgattt 1200

agaggagtta gtcttgaagt catgcgccgg ttaaggctaa actgaaagga caagttttgg 1260

tgactgcgct cctccaagcc agttacctcg gttcaaagag ttggtagctc agagaacctt 1320

cgaaaaaccg ccctgcaagg cggttttttc gttttcagag caagagatta cgcgcagacc 1380

aaaacgatct caagaagatc atcttattaa tcagataaaa tatttctaga tttcagtgca 1440

atttatctct tcaaatgtag cacctgaagt cagccccata cgatataagt tgtaattctc 1500

atgtttgaca gcttatcatc gataagcttt aatgcggtag tttatcacag ttaaattgct 1560

aacgcagtca ggcaccgtgt atgaaatcta acaatgcgct catcgtcatc ctcggcaccg 1620

tcaccctgga tgctgtaggc ataggcttgg ttatgccggt actgccgggc ctcttgcggg 1680

atatcgtcca ttccgacagc atcgccagtc actatggcgt gctgctagcg ctatatgcgt 1740

tgatgcaatt tctatgcgca cccgttctcg gagcactgtc cgaccgcttt ggccgccgcc 1800

cagtcctgct cgcttcgcta cttggagcca ctatcgacta cgcgatcatg gcgaccacac 1860

ccgtcctgtg gatcctctac gccggacgca tcgtggccgg catcaccggc gccacaggtg 1920

cggttgctgg cgcctatatc gccgacatca ccgatgggga agatcgggct cgccacttcg 1980

ggctcatgag cgcttgtttc ggcgtgggta tggtggcagg ccccgtggcc gggggactgt 2040

tgggcgccat ctccttgcat gcaccattcc ttgcggcggc ggtgctcaac ggcctcaacc 2100

tactactggg ctgcttccta atgcaggagt cgcataaggg agagcgtcga ccgatgccct 2160

tgagagcctt caacccagtc agctccttcc ggtgggcgcg gggcatgact atcgtcgccg 2220

cacttatgac tgtcttcttt atcatgcaac tcgtaggaca ggtgccggca gcgctctggg 2280

tcattttcgg cgaggaccgc tttcgctgga gcgcgacgat gatcggcctg tcgcttgcgg 2340

tattcggaat cttgcacgcc ctcgctcaag ccttcgtcac tggtcccgcc accaaacgtt 2400

tcggcgagaa gcaggccatt atcgccggca tggcggccga cgcgctgggc tacgtcttgc 2460

tggcgttcgc gacgcgaggc tggatggcct tccccattat gattcttctc gcttccggcg 2520

gcatcgggat gcccgcgttg caggccatgc tgtccaggca ggtagatgac gaccatcagg 2580

gacagcttca aggatcgctc gcggctctta ccagcctaac ttcgatcact ggaccgctga 2640

tcgtcacggc gatttatgcc gcctcggcga gcacatggaa cgggttggca tggattgtag 2700

gcgccgccct ataccttgtc tgcctccccg cgttgcgtcg cggtgcatgg agccgggcca 2760

cctcgacctg aatggaagcc ggcggcacct cgctaacgga ttcaccactc caagaattgg 2820

agccaatcaa ttcttgcgga gaactgtgaa tgcgcaaacc aacccttggc agaacatatc 2880

catcgcgtcc gccatctcca gcagccgcac gcggcgcatc tcgggcagcg ttgggtcctg 2940

gccacgggtg cgcatgatcg tgctcctgtc gttgaggacc cggctaggct ggcggggttg 3000

ccttactggt tagcagaatg aatcaccgat acgcgagcga acgtgaagcg actgctgctg 3060

caaaacgtct gcgacctgag caacaacatg aatggtcttc ggtttccgtg tttcgtaaag 3120

tctggaaacg cggaagtccc ctacgtgctg ctgaagttgc ccgcaacaga gagtggaacc 3180

aaccggtgat accacgatac tatgactgag agtcaacgcc atgagcggcc tcatttctta 3240

ttctgagtta caacagtccg caccgctgtc cggtagctcc ttccggtggg cgcggggcat 3300

gactatcgtc gccgcactta tgactgtctt ctttatcatg caactcgtag gacaggtgcc 3360

ggcagcgccc aacagtcccc cggccacggg gcctgccacc atacccacgc cgaaacaagc 3420

gccctgcacc attatgttcc ggatctgcat cgcaggatgc tgctggctac cctgtggaac 3480

acctacatct gtattaacga agcgctaacc gtttttatca ggctctggga ggcagaataa 3540

atgatcatat cgtcaattat tacctccacg gggagagcct gagcaaactg gcctcaggca 3600

tttgagaagc acacggtcac actgcttccg gtagtcaata aaccggtaaa ccagcaatag 3660

acataagcgg ctatttaacg accctgccct gaaccgacga ccgggtcgaa tttgctttcg 3720

aatttctgcc attcatccgc ttattatcac ttattcaggc gtagcaccag gcgtttaagg 3780

gcaccaataa ctgccttaaa aaaattacgc cccgccctgc cactcatcgc agtactgttg 3840

taattcatta agcattctgc cgacatggaa gccatcacag acggcatgat gaacctgaat 3900

cgccagcggc atcagcacct tgtcgccttg cgtataatat ttgcccatgg tgaaaacggg 3960

ggcgaagaag ttgtccatat tggccacgtt taaatcaaaa ctggtgaaac tcacccaggg 4020

attggctgag acgaaaaaca tattctcaat aaacccttta gggaaatagg ccaggttttc 4080

accgtaacac gccacatctt gcgaatatat gtgtagaaac tgccggaaat cgtcgtggta 4140

ttcactccag agcgatgaaa acgtttcagt ttgctcatgg aaaacggtgt aacaagggtg 4200

aacactatcc catatcacca gctcaccgtc tttcattgcc atacg 4245

<210> 23
<211> 33
<212> DNA
<213> Artificial Sequence

<220>
<223> Primer

<400> 23
gcatcatgag taaaaaaatt aatggatttg agg 33

<210> 24
<211> 35
<212> DNA
<213> Artificial Sequence

<220>
<223> Primer

<400> 24
ggtagatctt tagctattta ctaatgaaaa tttaa 35

<210> 25
<211> 33
<212> DNA
<213> Artificial Sequence

<220>
<223> Primer

<400> 25
gcaacatgtt aaaaaatgat tttaacgtat tgg                                    33

<210> 26
<211> 32
<212> DNA
<213> Artificial Sequence

<220>
<223> Primer

<400> 26
ggtagatctt tattgtagag ctgttattaa ct                                     32

<210> 27
<211> 35
<212> DNA
<213> Artificial Sequence

<220>
<223> Primer

<400> 27
gcatcatgag ttataaaaat gttaaaaatt taaca                                  35

<210> 28
<211> 33
<212> DNA
<213> Artificial Sequence

<220>
<223> Primer

<400> 28
ggtagatctt tatcctctta caattttttt gtg                                    33

<210> 29
<211> 34
<212> DNA
<213> Artificial Sequence

<220>
<223> Primer

<400> 29
gcatctagaa aatcataaaa aatttatttg cttt                                34

<210> 30
<211> 34
<212> DNA
<213> Artificial Sequence

<220>
<223> Primer

<400> 30
agctcgcgat tagctattta ctaatgaaaa ttta                                34

<210> 31
<211> 34
<212> DNA
<213> Artificial Sequence

<220>
<223> Primer

<400> 31
gcaatcgata aatcataaaa aatttatttg cttt                                34

<210> 32
<211> 32
<212> DNA
<213> Artificial Sequence

<220>
<223> Primer

<400> 32
agctcgcgat tattgtagag ctgttattaa ct                                  32

<210> 33
<211> 34
<212> DNA
<213> Artificial Sequence

<220>
<223> Primer

<400> 33
gcatctagaa aatcataaaa aatttatttg cttt                                34

<210> 34
<211> 32
<212> DNA
<213> Artificial Sequence

<220>

<223> Primer

<400> 34
agctcgcgat tatcctctta caattttttt gt                                    32